# EUROPEAN PATENT APPLICATION

(11) **EP 4 821 536 A2**
(43) Date of publication of application: **14.10.2026**
(21) Application number: 26200686.9
(22) Date of filing: 30.07.2024
(51) Int. Cl.: A61M 25/01

(54) **A CATHETER ASSEMBLY**

(30) Priority: 31.07.2023 US 202363516571 P; 31.07.2023 US 202363516566 P; 31.07.2023 US 202363516561 P; 20.09.2023 GB 202314379; 20.09.2023 GB 202314381; 20.09.2023 GB 202314382
(62) Document number(s) of the earlier application(s) in accordance with Art. 76 EPC: 24755330.8 / 4 746 949
(71) Applicant: ConvaTec Limited, Deeside, Flintshire CH5 2NU (GB)
(72) Inventor: PUPECK, Jay, Deeside, CH5 2NU (GB); PILMER, Nicola, Deeside, CH5 2NU (GB); URQUHART, Zac, Deeside, CH5 2NU (GB); DUHIG, Kate, Deeside, CH5 2NU (GB); PIASHEVICH, Aliaksandr, Deeside, CH5 2NU (GB); NOVAK, Marian, Deeside, CH5 2NU (GB); KANDRAC, Lukas, Deeside, CH5 2NU (GB)
(74) Representative: Wilson Gunn

(57) **Abstract**

A catheter assembly comprises a catheter, a funnel, and a tubular sleeve. The wetting mechanism and funnel are provided at either end of the sleeve and the wetting mechanism, funnel and sleeve together define a cavity containing the catheter. The wetting mechanism comprises a storage chamber configured to retain and release a wetting agent to wet the catheter in use. The funnel and wetting mechanism fit together to close the cavity with a push-fit. The catheter and storage chamber may be spaced from one another along the wetting mechanism. The wetting mechanism may be configured to simultaneously engage the inside and outside of the funnel. The wetting mechanism may comprise one or more tabs to engage the funnel. The funnel may comprise locating protrusions to engage the wetting mechanism. A cap may be provided and may fit into the same part of the wetting mechanism as the funnel.

## Description

### Technical Field of the Invention

The present invention relates to catheter assemblies, in particular to urinary catheter assemblies. Most particularly, but not exclusively, the invention relates to intermittent male urinary catheter assemblies, especially "open" catheter assemblies.

### Background to the Invention

Many people suffer from urinary problems that can make it difficult to pass urine. Intermittent catheter assemblies can provide a convenient and portable solution to this problem as a user is able to self-catheterise to relieve themselves as required. This reduces the effect of their urinary condition on their life and allows thm to enjoy a relatively normal lifestyle.

Known urinary catheter assemblies comprise funnels to direct the flow of liquid out of the catheter in use. For example, the funnel may be used to direct liquid from the catheter into a toilet bowl or other suitable receptacle. This can make the catheter assembly easier and more hygienic to use as the user is less likely to spill urine or have it contact their hands or clothing. Where a catheter assembly is used in this way without collecting the urine, it is known as a "open" catheter assembly.

Existing funnels for catheter assemblies need to be open-ended to ensure that liquid can flow freely out of them. However, this presents problems after use of the catheter because any residual liquid that remains in or around the catheter can leak out of the funnel. This causes a hygienic risk to the user as well as being inconvenient and causing discomfort or potential embarrassment.

One solution to this problem is provided in WO2016/206701A1 which discloses a catheter assembly comprising a catheter, a sleeve, a handle and a connector. The handle and connector are at either end of the sleeve and can be attached to one another to form a closed configuration wherein they completely encapsulate the catheter in a cavity formed by the sleeve and prevent drainage from the cavity. The handle and connector are provided with interlocking means such as a snap-fit means or a threaded coupling. The catheter assembly is provided in the closed configuration with a free liquid activating medium in the sleeve to render the catheter lubricious and ready for use. Some problems with this design are that the catheter assembly can be difficult to open and close due to the snap-fit or threaded interlocking means.

Existing funnels are also shaped to maximise production efficiency by using simple cylindrical shapes that are optimised for injection moulding. However, this means that they are often not easy for a user to handle and are specific to a type/size of catheter.

It is an object of embodiments of th present invention to at least partially overcome or alleviate the above problems and/or to provide an improved packaged catheter assembly.

### Summary of the Invention

The present invention provides a catheter assembly according to the appended claims.

In broad terms, the invention relates to a funnel for a catheter. The funnel may be configured to direct fluid out of a distal end of the catheter. The funnel may comprise a tube defining an internal profile of the funnel. The funnel may comprise a plurality of projections extending from the tube, preferably the projections extend outward from the tube. Each projection may comprise a tip distal from the tube. The tips of the projections may together define at least part of an external profile of the funnel. The internal profile may comprise a stepped region. A corresponding region of the external profile to the stepped region may be smooth.

According to a first aspect of the present invention there is provided a funnel for a catheter, the funnel being configured to direct fluid out of a distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth.

The funnel of the first aspect may include any one or more of the features of a funnel as described in general terms or in relation to the other aspects of the invention below.

The invention also relates to a catheter assembly comprising a catheter and a funnel. The catheter may comprise a proximal end for insertion into the body and a distal end. The funnel may be attached to the catheter, preferably to the distal end of the catheter. The funnel may be configured to direct liquid flow out of the catheter.

According to a second aspect of the present invention there is provided a catheter assembly comprising a catheter comprising a proximal end for insertion into the body and a distal end, and a funnel configured to direct fluid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth.

The external profile may therefore be substantially different from the internal profile. This allows additional design freedom to optimise the internal and external profiles for their different functions. In addition, due to the projections, the funnel is able to be manufactured cheaply and easily using injection moulding and without suffering from sink mark artefacts associated with injection moulding of objects with varying wall thickness. The projections also reduce the total material required reducing weight and cost. This also provides a more pleasant experience for the user when manipulating the funnel while the internal profile is stepped. This allows the same funnel to be used for different types/sizes of catheter. This means manufacturing is cheaper and simpler as less types of funnel need to be produced for all the different types/sizes of catheter.

The catheter assembly may comprise a sleeve. The sleeve may be configured to enclose at least part of the catheter from the proximal end to the distal end. The sleeve may be configured to enclose the majority of the catheter from the proximal end to the distal end. The sleeve may be configured to enclose substantially all of the catheter from the proximal end to the distal end.

The funnel may comprise a closure element. The closure element may be configured to inhibit fluid flow through, and/or out of, the funnel. In an open configuration fluid flow through, and/or out of, the funnel may be permitted. In a closed configuration fluid flow through, and/or out of, the funnel may be inhibited. The closure element may be moveable from the open configuration to a closed configuration. The fluid is preferably a liquid, for example urine. The closure element may be connected to the funnel in the open configuration. The closure element may be connected to the funnel in the closed configuration. Preferably, the closure element is connected to the funnel in both the open and closed configurations.

Accordingly in another aspect of the invention there is provided a funnel for a catheter, the funnel comprising a closure element configured to inhibit liquid flow out of the funnel, wherein the closure element is moveable from an open configuration in which liquid flow out of the funnel is permitted to a closed configuration in which fluid flow out of the funnel is inhibited, wherein the closure element is connected to the funnel in both the open and closed configurations. This makes the closure element more convenient and easy to use.

Similarly, another aspect provides a catheter assembly comprising a catheter comprising a proximal end for insertion into the body and a distal end, and a funnel configured to direct liquid out of the distal end of the catheter, the funnel comprising a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations.

Advantageously, the catheter assembly is thereby easier to use as the closure element is conveniently connected to the funnel and is therefore less likely to be misplaced or lost during use. In addition, as it is part of the funnel, it is ideally located to close the end of the funnel and inhibit liquid flow through it without requiring manipulation of the rest of the catheter assembly such as the catheter or sleeve.

The closure element may be provided in, and/or moveable to, a stowed position. In the stowed position, the closure element may not impede liquid flow out of the funnel. When the closure element is in the stowed position, the catheter assembly may thus be in the open configuration. Thus, in the stowed position, the closure element may be completely out the way of liquid as it flows out the funnel in use. It is therefore less likely to contact the liquid during use which can make subsequent use of the closure element unpleasant and difficult as well as presenting a hygienic risk to the user.

In the stowed position, liquid flowing out of the funnel may not contact the closure element. In the stowed position, the closure element may be positioned outside a liquid flow path out of the funnel. The funnel may comprise an axis which defines a liquid flow path through the funnel. The liquid flow path may be defined outside the funnel with a conical shape diverging from the liquid flow path through the funnel with a given aperture half-angle. The aperture half angle may be no more than 60 degrees, 45 degrees, 30 degrees, 20 degrees or 10 degrees. The aperture half angle may be at least 10 degrees, 20 degrees, 30 degrees, 45 degrees or 60 degrees. Preferably, the aperture half angle is about 30 degrees. Thus, the closure element is stowed to ensure it is out of the liquid flow path.

The closure element may comprise a retainer. The retainer may be configured to retain the closure element in the stowed configuration. The retainer may be resiliently biased to urge the closure element into the stowed configuration. The retainer may attach part of the closure element to an outside of the funnel to retain it in the closed configuration. The retainer may attach two parts of the closure elements together to retain it in the closed configuration. Thus, the retainer ensures the closure element remains stowed until it is needed for use.

The retainer may be configured to release the closure element from the stowed position when actuated. The retainer may be user actuatable. The retainer may be breakable. Preferably, the retainer is breakable to release the closure element from the stowed position. Thus, the user can simply break the retainer in order to release the closure element.

The closure element may comprise a connector. The connector may be attached to the funnel. The connector may attach the closure element to the funnel. The connector may be flexible. The closure element may comprise a closure body. The closure body may be configured to inhibit liquid flow out of the funnel in the closed configuration. The connector may be configured to facilitate movement of the closure body with respect to the funnel. Thus, the closure element is provided with an intuitive design that is easy to use.

The connector may be configured to allow the closure body to rotate with respect to the funnel. The connector may comprise a flexible material. The connector may comprise a hinge. The retainer may be configured to inhibit movement of the closure body when in the stowed position. The retainer may be configured to inhibit movement of the connector when in the stowed position. The connector may be elongate with two ends. The connector may be attached at a first end to the funnel. The connector may be attached at a second end to the closure body. The retainer may attach the connector to the funnel at a point between the two ends of the connector. The retainer may attach the connector to the closure body at a point between the two ends of the connector. Preferably, the retainer comprises at least two retention links. Each retention link may be breakable. A first retention link may attach the connector to the closure body. A second retention link may attach the connector to the funnel. The first retention link may attach the connector to the closure body at a point on the connector that is closer to the first end than the point the second retention link attaches the connector to the funnel. Thus, the connector may be alternately connected to the funnel, then closure body, then funnel, then closure body. The connector may be substantially S-shaped in the stowed position. The closure element may be provided between the inlet and outlet end of the funnel in the stowed position. Preferably the closure element is completely between the inlet and outlet end in the stowed position. The closure element may not extend past the outlet end of the funnel in the stowed position. The closure element may be provided outside the funnel tube in the stowed position. This helps ensure the closure body is securely held against the funnel in the stowed position.

The closure element may comprise a plug. The plug may be configured to plug the funnel, for example in the closed configuration. The closure body may comprise the plug. The plug may be configured to at least partially block the funnel. Thus, the closure element may effectively inhibit liquid flow through and out of the funnel by plugging the funnel.

The plug may be shaped to match the internal profile of the funnel. The plug may be generally tubular. The plug may be cylindrical. The plug may be tapered. The plug may comprise an insertion end. The insertion end may be configured to be inserted into the funnel to plug it. The plug may taper down towards the insertion end. The plug may comprise a cap end distal from the insertion end. The cap end may be configured to prevent over-insertion of the plug into the funnel. The cap end may be sized to abut the end of the funnel. The connector may be attached to the closure body via the cap end. The plug may have a length measured from the cap end to the insertion end. The insertion end may define a tip of the plug. The plug may have a length of at least 10%, at least 20%, at least 30%, at least 40% or at least 50% the length of the funnel. The plug may have a length of no more than 60%, no more than 50%, no more than 40% or no more than 30% the length of the funnel. Preferably, the plug has a length of about 40% the length of the funnel. Thus, the plug is able to snuggly and efficiently plug the funnel.

The closure element may comprise one or more sealing protrusions. The funnel may comprise one or more sealing protrusions. The one or more sealing protrusions may be resiliently deformable to provide a seal against the funnel, and/or closure element, preferably between the funnel and closure element. The one or more sealing protrusions may extend from a surface of the closure element. Thus, the closure element can more effectively seal the funnel to ensure liquid is inhibited from flowing through and out of it.

The one or more sealing protrusions may comprise one or more sealing ribs. Each sealing rib may extend around the closure element, preferably completely around the closure element in a ring shape. The plug may comprise the one or more sealing ribs. The plug may comprise two or more sealing ribs, three or more sealing ribs or preferably four sealing ribs. The one or more sealing ribs may be spaced along the length of the plug. The one or more sealing ribs may comprise a tip rib. The tip rib may extend around the insertion end of the plug. The one or more sealing ribs may comprise a cap rib. The cap rib may be at or adjacent to the cap end. The cap rib may extend from the plug adjacent to the cap end, for example 10-20% of the distance along the plug from the cap end. The one or more sealing ribs may comprise one or more intermediate ribs. The intermediate ribs may be positioned at any position along the plug. The intermediate ribs may comprise a first intermediate rib. The first intermediate rib may be positioned towards the insertion end. The first intermediate rib may be positioned 20-30% of the length of the plug from the insertion end. The intermediate ribs may comprise a second intermediate rib. The second intermediate rib may be position towards the cap end. The second intermediate rib may be positioned approximately the same distance from the cap rib that the cap rib is from the cap end. The second intermediate rib may be positioned 20-40% of the length of the plug from the cap end. Preferably, the one or more sealing ribs comprise the tip rib, first and second intermediate ribs and the cap rib. Thus, the sealing ribs help ensure a tight and secure liquid seal between the plug and funnel.

The closure element may comprise a release mechanism. The release mechanism may be configured to facilitate movement of the closure mechanism out of the closed configuration. The release mechanism may be user actuatable. The release mechanism may comprise a tab. The tab may extend from the closure element. The tab may extend from the cap end. The tab may extend outside the funnel. The tab may comprise a semi-circular protrusion from the cap end. The tab may be positioned at an opposite side of the cap end from the connector. The cap end may be substantially flat on a side opposite from the insertion end. Thus, the closure element can be more easily disengaged from the funnel if necessary.

The closure element may comprise a cap. The cap may be configured to cap the funnel in the closed configuration. The cap may be configured to seal around the funnel to inhibit liquid flow through the funnel. The cap may be configured to snap-fit around the funnel. Thus liquid flow is effectively inhibited by the cap.

The closure element and funnel may be integrally formed. The closure element and funnel may be injection moulded. Thus, the closure element and funnel are cheaper and more efficient to form.

The funnel may be generally tubular. The funnel may be open ended. The funnel may therefore comprise a tube. The funnel may comprise an inlet end. The inlet end may be configured to be coupled to the catheter. The funnel may comprise an outlet end. The funnel may have a length measured between the inlet and outlet ends. The outlet end may be configured to allow liquid to pass out of the funnel in use. The closure element may be configured to block the outlet end to inhibit liquid flow out of the funnel. The closure element may plug the outlet end. The closure element may be configured to be at least partially inserted into the outlet end to inhibit liquid flow out of the funnel.

The funnel may comprise an outlet portion. The outlet portion may be configured to direct the flow of liquid out of the funnel. The outlet end may be provided in the outlet portion. The outlet portion may be generally tubular. The outlet portion may be tapered. The outlet portion may have a substantially constant wall thickness. The outlet portion may be frustoconical. The closure element may be attached to the outlet portion. The closure element may be attached to the outlet portion adjacent to the outlet end. The connector may be attached to the outlet portion adjacent to the outlet end.

The catheter may therefore be rotation dependent. The catheter may be configured to be inserted into the body with a specific orientation. The catheter may comprise an insertion orientation configured to enable the catheter to release fluid from the body. The insertion orientation may define a rotational orientation of the catheter along its axis. The funnel may comprise a locator. The locator may be configured to identify the orientation of the catheter. The locator may be positioned to correspond to a curve in the catheter. The locator may be provided where the catheter is curved, for example where the catheter is a coudé catheter. The coudé catheter may comprise a curve at or adjacent to the proximal end. Thus, the locator assists the user in using the catheter and aligning it with their bladder once inside the body.

The closure element may be positioned to one side of the locator. The closure element may be connected to the funnel on one side of the locator. The closure element may be arranged above or to one side of the funnel (for example above, or to the left or right, and not directly below the funnel) when the catheter is in the insertion orientation. This helps ensure the closure element is to one side of liquid as it passes out of the funnel.

The locator may be positioned upwards in use. The closure element is preferably not substantially opposite the locator rib on the funnel. This helps ensure drips of liquid from the funnel do not run out the funnel onto the closure element.

The closure element is preferably spaced from the locator. The closure element may be spaced by at least 30 degrees, at least 50 degrees, at least 70 degrees, or 90 degrees around the circumference of the funnel from the locator. The closure element may be spaced by no more than 90 degrees, no more than 70 degrees, no more than 50 degrees, or no more than 30 degrees around the circumference of the funnel from the locator. The closure element is preferably not at the same or a corresponding position as the locator. This helps ensure the locator rib is easy to identify in use.

The locator may comprise a locator rib. The locator rib may extend along at least part of the length of the funnel. The locator may comprise a locator marking.

The funnel may comprise an inlet portion. The inlet end may be provided in the inlet portion. The outlet portion may be joined to the inlet portion. The outlet portion may be narrowest at the point it meets the inlet portion. The inlet portion may be narrowest at the point it meets the outlet portion. The outlet portion may cover at least 40%, at least 50%, at least 60%, at least 70% or at least 80% the length of the funnel. The outlet portion may cover no more than 80%, no more than 70%, no more than 60%, or no more than 50% the length of the funnel. Preferably the outlet portion covers 60-70% the length of the funnel. Preferably the outlet portion is longer than the inlet portion.

The inlet portion may be configured to receive the catheter. The inlet portion may be configured to provide a liquid path from the inside of the catheter into the funnel. The inlet portion may be configured to engage the catheter. The inlet portion may be configured to receive the distal end of the catheter. Thus, the catheter may be securely attached to the funnel.

As mentioned above, the funnel may comprise a tube. The tube may define the internal profile of the funnel. The tube may define at least part of the external profile of the funnel. The funnel may comprise a plurality of protrusions. The protrusions may extend from the tube. The protrusions may extend outward from the tube. Each protrusion may comprise a tip distal from the tube. The tips of the protrusions may together define at least part of an external profile of the funnel.

The internal profile of the funnel may comprise a stepped region. A region of the external profile corresponding to the stepped region may be smooth. The external profile may therefore be substantially different from the internal profile. This allows additional design freedom to optimise the internal and external profiles for their different functions. While a stepped region is given as an example, the internal profile may alternatively be chamfered, tapered or have a different irregular shape, and a corresponding part of the external profile be smoothed or otherwise have a different shape as defined by the protrusions.

In addition, due to the projections, the funnel is able to be manufactured cheaply and easily using injection moulding and without suffering from sink mark artefacts associated with injection moulding of objects with varying wall thickness. The projections also reduce the total material required reducing weight and cost. This also provides a more pleasant experience for the user when manipulating the funnel while the internal profile is stepped. Due to the provided design freedom to alter the internal profile independently of the external profile, the same funnel to be used for different types/sizes of catheter. This means manufacturing is cheaper and simpler as less types of funnel need to be produced for all the different types/sizes of catheter.

A plurality of gaps may be provided between the plurality of projections. The external profile of the funnel may extend across the gaps between the tips of the protrusions. Thus, when gripping the funnel, the funnel is more easy to grip because the alternate gaps and protrusions provide a undulating surface. However, the appearance and feel of the funnel remains enhanced due to the overall external profile being defined by the tips of the protrusions.

The tube may have a substantially constant wall thickness. The projections may have a wall thickness that is substantially the same as the wall thickness of the tube. The wall thickness of the tube may be at least 0.5 mm, at least 0.7 mm, or at least 1 mm. The wall thickness of the projections may be no more than 1.5 mm, no more than 1 mm, no more than 0.7 mm. The wall thickness of the projections may be at least 0.5 mm, at least 0.7 mm, or at least 1 mm. The wall thickness of the tube may be no more than 1.5 mm, no more than 1 mm, no more than 0.7 mm. Thus, the wall thicknesses are the same which means that the funnel can be effectively manufactured with injection moulding and without sink mark artefacts.

The projections may be provided at or adjacent to the inlet end of the funnel. The projections may be provided spaced apart in the inlet portion of the funnel. The projections may define the external profile of the funnel in the inlet portion. The projections may define the external profile of the funnel for substantially all of the inlet portion. Thus, the projections are positioned where the internal profile needs to change to accommodate connections to the catheter.

The stepped region may comprise at least two steps in the internal profile. Thus, the internal profile may be sized to fit at least two different types/sizes of catheter. The projections may define the external profile of the funnel at the two steps and preferably also all the way between them. To achieve this, the projections may be provided in positions along the funnel corresponding to the two steps, and preferably, the space between the two steps. Thus, the projections help ensure the funnel has a smooth outer profile while accommodating more complex internal shapes.

The stepped profile may comprise a transition point at a step in the internal profile. The projections may be provided on one side of the transition point, or both sides. The projections may be provided on the side of the transition point that corresponds to the side of the transition point where the internal profile is narrower and/or smaller side. Thus, by providing projections on one side of the transition point they can be used to counteract the effect of the step in the internal profile.

The projections may be provided across the transition point, for example on both sides of the transition point. The projections may extend further from the tube on one side of the transition point than the other. A corresponding but substantially inverse change in the projections may be provided at the transition point as compared to the change in the internal profile at the transition point. Thus, the size/shape of the projections is varied across the transition point to ensure that the effect of the step in the internal profile is less prominent, smoothed and/or indistinguishable in the external profile.

The external profile defined by the tips may change along the length of the funnel. This is particularly beneficial when a specific external shape is required without affecting the internal profile defined by the tube. This can allow the creation of funnels with an external profile that is more suited to manual manipulation by the user and especially for users with limited dexterity.

The projections may have a radial length defined as the distance the projections extend from the internal tube in a radial direction. The radial length may change along the length of the funnel. The radial length of two adjacent projections may be different. Changes in the radial length may counter changes in the internal profile. The radial length may be at least 1 mm, at least 1.5 mm, at least 2mm. The radial length may be no more than 3 mm, no more than 2 mm or no more than 1.5 mm. Thus, the length of the projections can be easily adjusted to ensure the correct external profile for the funnel irrespective of the internal profile.

The external profile may diverge in a direction from the outlet end towards the inlet end. The external profile may curve along the length of the funnel. The external profile may be concave. The external profile may comprise a curved region. The external profile may curve outward towards the catheter/inlet end. The external profile may diverge outward along the inlet portion of the funnel. The external profile may diverge along at least 5 mm, at least 7 mm, at least 10 mm, at least 15 mm of the length of the funnel. The external profile may diverge along no more than 15 mm, no more than 10 mm, no more than 7 mm or no more than 5 mm of the length of the funnel. The external profile may diverge outward from the outlet portion towards the inlet end. The external profile as described in this paragraph is preferably defined by the tips of the protrusions. Thus, the funnel is shaped to be more easily gripped and is curved so that it is easier to apply a force along the length of the funnel to move the catheter into/out of the body.

The external profile may be substantially constant along a part of the funnel. The external profile may be substantially constant adjacent to, and/or extending from, the inlet end. The external profile may be substantially constant over along at least 5 mm, at least 7 mm, at least 10 mm, at least 15 mm of the length of the funnel. The external profile may be substantially constant along no more than 15 mm, no more than 10 mm, no more than 7 mm or no more than 5 mm of the length of the funnel. The external profile as described in this paragraph is preferably defined by the tips of the protrusions. The external profile as defined by the tips of the protrusions is preferably widest where the external profile is substantially constant. This helps ensure there are no sharp edges on the external profile. The external profile along the inlet portion is preferably widest where the external profile is substantially constant. The internal profile may be tapered where the external profile is constant. The external profile may be constant adjacent to the inlet end.

The external profile along the inlet portion may be defined by the tips of the protrusions. The external profile along the outlet portion may be defined by the tube. The protrusions may be provided along the inlet portion. The protrusions are preferably only provided along the inlet portion. The internal profile of the funnel may comprise a flange where the outlet portion meets the inlet portion. The flange may extend between the outlet portion and the inlet portion. The flange may define a step change in the internal profile of the funnel. The external profile of the funnel may be substantially constant across the flange from the outlet portion to the inlet portion. Thus the changes in the size of the internal profile where the inlet and outlet portions meet are not translated into the external profile due to the protrusions.

The flange may comprise an inner perimeter (or diameter where circular) where it meets the smaller of the inlet portion or outlet portion. The flange may comprise an outer perimeter (or diameter where circular) where it meets the larger of the inlet portion or outlet portion. The outlet portion may extend around the inlet portion. The outlet portion is preferably larger than the inlet portion at the flange. The outer perimeter may be at least 3 mm, at least 4 mm, or at least 5 mm wider than the internal perimeter. The outer perimeter may be no more than 6 mm, no more than 5 mm, or no more than 4 mm wider than the internal perimeter. The inner perimeter may correspond to the internal size of the smallest catheter intended to be fitted to the funnel. The inner perimeter may have a diameter of no more than 6 mm, no more than 5 mm, no more than 4 mm or no more than 3 mm. The inner perimeter may have a diameter of at least 2 mm, at least 3 mm, at least 4 mm or at least 5 mm. This helps ensure efficient liquid flow from the catheter and into the funnel.

The funnel may be configured to receive an external connector device. The outlet end may be configured to receive the external connector device. The outlet portion may be configured to receive the external connector device. The flange may comprise a sealing ring. The sealing ring may extend from the flange towards the outlet end. The sealing ring may be configured to provide a seal with the external connector device. The sealing ring may be configured to abut the external connector device. Thus, the funnel can be easily and conveniently connected to other devices, for example where a sample of the liquid passing through the catheter is required.

The tube may comprise two or more catheter receiving zones, preferably only two catheter receiving zones. Each catheter receiving zone may be configured to receive and retain a different type and/or size of catheter. Each catheter receiving zone may be sized to correspond to the external profile of a specific type and/or size of catheter. Each catheter receiving zone is preferably configured to correspond to the size/shape of a specific urinary catheter, most preferably a male urinary catheter. The two or more catheter receiving zones may be arranged in series along the length of the funnel. The two or more catheter receiving zones may be arranged on the same axis, or coaxially. Thus, the two or more receiving zones allow the funnel to be used on multiple types/sizes of catheter. This reduces the number of different funnels which need to be manufactured, reducing the complexity of making a range of catheters with funnels. In addition, as they are provided in series, then the process of manufacturing a catheter assembly is simplified as any catheter that fits the funnel can be simply inserted into it and fitted in the same hole. Of course, in some embodiments, the funnel may only comprise one catheter receiving zone.

The internal profile of the funnel may change between adjacent catheter receiving zones. The internal profile may be stepped between adjacent catheter receiving zones. The stepped region may span at least two catheter receiving zones. A catheter stop may be provided at an end of each catheter receiving zone distal from the inlet end. The catheter stop may be provided in the stepped region. The catheter stop may be configured to prevent over insertion of a catheter into the funnel. The catheter stop may be configured to prevent over insertion of a catheter into a corresponding catheter receiving zone. The catheter stop may comprise a catheter flange. Thus by providing a catheter flange, the catheter is better retained in the funnel without unnecessarily deforming the catheter which could lead to a reduction of liquid flow through the catheter.

The projections may be provided in a position corresponding to at least one of the two or more catheter receiving zones. The projections may be provided in a position corresponding to at least two of the two or more catheter receiving zones. The projections may span all the way between at least two of the two or more catheter receiving zones. The projections may be provided in a position corresponding to each of the two or more catheter receiving zones. The projections may span all the way between each of the two or more catheter receiving zones. Thus, the projections ensure the external profile remains smooth and consistent while the internal profile changes to accommodate the catheter receiving zones.

The internal profile may be tapered adjacent to the inlet end. The internal profile may be converging from the inlet end towards the outlet portion. The internal profile may taper inwards from the inlet end to the catheter receiving zone or catheter receiving zones. The inlet end may have an internal diameter of at least 3 mm, at least 4 mm, at least 5 mm, at least 6 mm or at least 7 mm. The inlet end may have an internal diameter of no more than 8 mm, no more than 7 mm, no more than 6 mm, no more than 5 mm. Preferably, the inlet end has an internal diameter of about 6 mm or about 5 mm. The catheter receiving zone proximal to the inlet end may have a diameter that is about 0.5 to 1.5 mm smaller than the inlet end, for example 0.7 mm smaller. Thus, when fitting a catheter to the funnel, the taper helps guide the catheter into the catheter receiving zone or zones to be retained by the funnel.

Each projection may be tapered. Each projection may taper down towards the tip. Each projection may be narrowest and/or smallest at the tip. This helps ensure even injection moulding and make it easier to remove the funnel from the mould once formed.

The tip of each projection may have an outer face defining the external profile of the funnel. The tip of each projection may provide a smooth outer face. The tip of each projection may have a substantially planar external face. Thus, the tips are smooth and together provide an external profile that is generally comfortable to hold whilst being easy to grip due to the gaps between the protrusions.

The plurality of projections may comprise one or more ribs. Each rib may be elongate and extend around and/or along the funnel. Each rib may have a length measured along the rib as it extends around and/or along the funnel. Each rib may have a thickness defined perpendicular to its length. The thickness of each rib may be substantially the same as the wall thickness of the tube. Each rib may have a thickness of between 0.5 to 3 mm, or preferably between 0.5 to 1.5 mm. The thickness of each rib may be tapered. The thickness of each rib may be smallest at its tip. The ribs thus provide a stronger structure to the protrusions while helping to ensure even injection moulding and making it easier to remove the funnel from the mould once formed.

The one or more ribs may comprise two or more ribs. At least two of the two or more ribs may be intersecting ribs. The intersecting ribs may intersect one another at substantially perpendicular angles. Thus, the ribs are able to provide structural support to one another and also facilitate the creation of a more easily gripped funnel that can provide good gripping action in multiple directions.

The one or more ribs may comprise one or more circumferential ribs extending around a circumference of the funnel. The circumferential ribs may extend at least 20%, at least 30%, at least 40%, at least 50%, at least 60%, at least 70%, at least 80%, at least 90% or at least 100% of the way around the circumference of the funnel. The circumferential ribs may be evenly spaced along the length of the funnel. The funnel may comprise at least five, at least six, at least seven, at least eight, at least nine, at least ten circumferential ribs. The funnel may comprise no more than fifteen, no more than fourteen, no more than thirteen, no more than twelve, no more than eleven, no more than ten circumferential ribs. Thus, the circumferential ribs help to ensure the funnel is easy to grip and apply a force along the length of the funnel to move the catheter into and out of the body.

A circumferential rib may be provided around the inlet end. The circumferential rib around the inlet end may provide an inlet flange. An outer diameter of the inlet flange may be at least 8 mm, at least 10 mm, or at least 12 mm. An outer diameter of the inlet flange may be no more than 15 mm, no more than 12 mm or no more than 10 mm. Preferably, the outer diameter of the inlet flange is about 10 mm. Thus a flange is provided around the inlet end to help ensure the funnel is easier to use and manufacture as part of a catheter assembly.

The one or more ribs may comprise one or more axial ribs extending along the length of the funnel. The radial length of an axial rib may change along the length of the funnel. The one or more axial ribs may be evenly spaced around the circumference of the funnel. The one or more ribs may comprise at least four ribs. The at least four ribs may comprise at least four axial ribs. Each axial rib may intersect at least two other ribs, preferably at least two circumferential ribs. Each axial rib may extend along the majority of the length of the funnel covered by the protrusions. Each axial rib preferably extends along the entire length of the funnel that is covered by the protrusions. Each axial rib may extend between the outlet portion to the inlet end. Each axial rib may extend the whole way between the outlet portion to the inlet end, e.g. across the whole of the inlet portion. Each axial rib may be substantially the same length. Thus, the axial ribs provide structural support to the circumferential ribs.

The funnel may be formed of a plastics material. The funnel may be relatively rigid compared to the catheter and/or sleeve. The funnel may be formed from any suitable material, for example polyethylene, or preferably linear low-density polyethylene (LLDPE).

The catheter assembly may further comprise a wetting mechanism. The wetting mechanism may be formed of a plastics material. The wetting mechanism may be relatively rigid compared to the catheter, sleeve and funnel. The wetting mechanism may be formed from any suitable material, for example polyethylene, polypropylene (PP) and/or linear low-density polyethylene (LLDPE). The wetting mechanism may be formed of the same material as the funnel. The wetting mechanism may be structurally more rigid than the funnel. The wetting mechanism may be thicker than the funnel, for example it may have a larger effective wall thickness. The wetting mechanism may be tubular. The wetting mechanism may have a length defined along the tubular shape of the wetting mechanism.

The wetting mechanism may be configured to store a wetting agent. The wetting mechanism may be configured to release the wetting agent to wet the catheter in use. The wetting mechanism may be user actuatable to release the wetting agent to wet the catheter in use. The wetting mechanism may be configured to be user actuatable to independently release the wetting agent to wet the catheter. Thus, the wetting mechanism may be configured to independently release wetting agent when actuated by a user. For example, the wetting mechanism may be able to release wetting agent without user actuation of the other parts of the catheter assembly. The wetting mechanism may comprise an external housing. The wetting mechanism may comprise a cap. The external housing and cap may form a cavity for housing at least part of the catheter. The external housing may comprise a wetting agent storage chamber. The wetting mechanism may comprise an inserter. The wetting agent storage chamber may be defined by the housing and an inserter. The inserter may be moveable with respect to the housing between a first position and a second position. In the first position, the inserter may be retracted. In the first position, the inserter may be aligned with the housing to seal the storage chamber. In the second position, the inserter may be deployed. In the second position, the inserter may be misaligned with the housing to open the storage chamber. Removal of the cap from the external housing may move the inserter from the first position to the second position. Thus, the inserter may function as a sealing member and may be referred to as such. As such, in one example, the wetting mechanism comprises a housing and a sealing member that together define a wetting agent storage chamber, wherein the sealing member is moveable with respect to the housing between a first position and a second position, wherein in the first position, the sealing member is aligned with the housing to seal the storage chamber, and in the second position, the sealing member is misaligned with the housing to open the storage chamber and release the wetting agent. Thus, the sealing member is preferably a part of the wetting mechanism. It is therefore not provided by the catheter. This helps provide a selectively openable wetting mechanism that can be independently actuated at a convenient time for the user.

The wetting mechanism may be positioned at one end of the sleeve. The wetting mechanism may be provided at an end of the sleeve distal from the funnel. The wetting mechanism, sleeve and funnel may provide a cavity containing the catheter. The wetting mechanism may be configured to release the wetting agent into the sleeve to wet the catheter. The catheter may be provided in a position spaced apart from the storage chamber, preferably spaced apart along the length of the wetting mechanism. The proximal end of the catheter may be spaced part from the storage chamber along the length of the wetting mechanism. This helps ensure the wetting agent released into the sleeve effectively wets the entire catheter and especially the proximal end.

The housing may comprise a catheter end. The catheter end may be coupled to the sleeve. The housing may comprise a cap end. The cap end may be configured to receive the cap. The housing may comprise a neck. The neck may extend from the sleeve. The housing may have a length defined from the catheter end to the cap end. The neck may cover approximately one third the length of the housing. The housing may comprise a divergent portion. The divergent portion may cover about one quarter the length of the housing. The neck may extend from the catheter end to the divergent portion. The radial size of the housing may increase over the divergent portion. The housing may comprise a chamber wall. The chamber wall may extend from the divergent portion to the cap end.

The neck may define a narrowest part of the internal profile of the housing. The internal profile may be substantially constant along the neck. The neck may have a diameter approximately double the diameter of the catheter. The chamber wall may define a widest part of the internal profile of the housing. The internal profile may be substantially constant along the chamber wall. The chamber wall may have a diameter approximately double the diameter of the neck. The divergent portion may have an internal profile that is substantially constant over a majority of the divergent portion. The diameter of the constant part of the internal profile of the divergent portion is preferably about halfway between the diameter of the neck and chamber wall. The divergent portion may have a stepped, tapered, or chamfered region where it meets the neck. The divergent portion may have a stepped, tapered, or chamfered region where it meets the chamber wall.

The housing may have a substantially constant wall thickness. The external profile of the housing may be substantially the same as the internal profile. The external profile of the housing may comprise one or more gripping ribs extending from the housing. A gripping rib may be provided around the cap end. A gripping rib may be provided between the chamber wall and divergent portion.

The inserter may be tubular. The inserter may fit partially within the housing. The housing and inserter may be concentrically arranged. The inserter may be radially nested within the housing. The inserter may comprise an inserter tip. The inserter tip may be provided at a first end of the inserter. The inserter tip may be configured to guide passage of the catheter into the body in use. The inserter tip may close the first end. The inserter tip may comprise a hemispherical end. The hemispherical end may be openable. The hemispherical end may comprise one or more apertures or openings such as slits which allow the end to be opened to allow passage of a catheter therethrough.

The inserter may comprise a catheter gripper. The catheter gripper may be provided at an opposite end from the inserter tip. The catheter gripper may be configured to grip the catheter. The catheter gripper may comprise a pair of gripping protrusions. The gripping protrusions may extend outward from the inserter. The gripping protrusions may be provided on opposite sides of the inserter. The gripping protrusions may be sized to fit within the internal diameter of the divergent portion. The gripping protrusions may be too large for the internal diameter of the neck. A pair of gripping slots may be provided. The gripping slots may be opposing. The gripping slots may be provided between the gripping protrusions. The gripping slots may be configured to allow the inserter to flex. The gripping slots may be configured to allow the gripping protrusions to fit within the neck. The gripping slots may be configured to allow the inserter to narrow to grip the catheter.

The inserter may comprise a first seal. The first seal may be configured to prevent wetting agent leaving the cap end of the housing. The first seal may be provided towards the cap end of the housing. The first seal may be configured to seal against the chamber wall. The inserter may comprise a second seal. The second seal may be configured to prevent wetting agent leaving the catheter end of the housing. The second seal may be provided towards the catheter end. The second seal may be configured to seal against the divergent portion.

The first seal may comprise a pair of ribs. The second seal may comprise a pair of ribs. Each of the pair of ribs may be mounted on a support structure, for example a trapezoid support structure.

The inserter may comprise a pair of activation tabs. The pair of activation tabs may be provided on opposing sides of the inserter. The activation tabs may be provided between the first seal and the inserter tip. The activation tabs may extend outward from the inserter. The activation tabs may extend around only part of the circumference of the inserter.

The cap may comprise a sheath. The sheath may be open-ended. The sheath may be configured to enclose the inserter tip. The sheath may comprise a closed end. The closed end may be hemispherical. The cap may comprise a pull ring. The pull ring may extend from the closed end of the cap. The pull ring may be sized to allow a user to insert their finger into the ring.

The cap may comprise an activation marker. The activation marker may indicate how to remove the cap. The activation marker may be an arrow.

The cap may comprise a pair of apertures. The apertures may be arranged to receive the activation tabs of the inserter. The cap may comprise a pair of slits. The slits may be provided on opposing sides of the cap and between the apertures. The slits may be arranged to allow the cap to flex. The slits may allow the apertures to engage and/or disengage from the activation tabs.

The cap may comprise a plurality of locator bumps. The locator bumbs may be provided in pairs around the circumference of the cap. The cap end may be configured to engage the locator bumps to locate the cap with respect to the housing. The cap end may be received between the two locator bumps in each pair of locator bumps.

The wetting mechanism may be formed of two different materials. The wetting mechanism may be formed from at least two component parts, for example at least five component parts. The at least two component parts may comprise the inserter. The at least two component parts may comprise the cap. At least two of the component parts may be formed of a different material. The housing may comprise at least two, or at least three, of the component parts. The housing may comprise a first component part and a second component part. The first component part may comprise polypropylene (PP). The first component part may be relatively rigid, for example compared to the funnel. The first component part may be configured to provide structural rigidity to the wetting mechanism. The first component part may form at least part of the chamber wall. The first component part may form at least part of the divergent portion. The first component part may define at least part of an internal profile of the wetting mechanism. The first component part may define at least part of an internal profile of the chamber wall. The first component part may define at least part of an internal profile of the divergent portion. The first component part thereby ensures the necessary rigidity of the housing.

The second component part may comprise linear low-density polyethylene (LLDPE). The second component part may be overmoulded onto the first component part. The second component part may form at least part of an external profile of the wetting mechanism. The second component part may form at least part of the chamber wall. The second component part may form at least part of the divergent portion. The second component part may form at least part of the neck. The second component part thereby helps to provide a more comfortable outer feel to the housing.

The housing may comprise a third component part. The third component part may form the cap end of the housing. The third component part may define part of the external profile of the chamber wall. The third component part may comprise polypropylene (PP). The third component part may be relatively rigid, for example compared to the funnel. The third component part may be configured to provide structural rigidity to the wetting mechanism. The third component part may be attach to the first component part. A locking mechanism may be provided to attach the first and third component parts together. The locking mechanism may comprise a snap-fit. The locking mechanism may comprise complementary locking slots/protrusions.

In the first position, the gripping protrusions may be arranged in the neck. In the first position, the catheter gripper may be compressed inward. In the first position, the catheter gripper may grip the catheter. In the first position, the second seal may be positioned in the divergent portion. In the first position, the second seal may seal between the inserter and the housing. In the first position, the first seal may be positioned in the chamber wall. In the first position, the first seal may seal between the inserter and the housing. In the first position, the activation tabs may be positioned inside the housing. In the first position, the cap may be inserted into the housing. In the first position, the apertures may receive the activation tabs. In the first position, the cap end may bear onto the cap between the locator bumps in each pair of locator bumps. In the first position, the cap end may prevent the cap from flexing outward. In the first position, the cap end may prevent removal of the cap from the wetting mechanism.

In the second position, the gripping protrusions may be arranged in the divergent portion. In the second position, the catheter gripper may not be compressed. In the second position, the catheter gripper may release the catheter. In the second position, the second seal may be positioned in the chamber wall. In the second position, a liquid path may be provided around the second seal. In the second position, a gap may be provided between the inserter and chamber wall. In the second position, the second seal may not seal between the inserter and chamber wall. In the second position, the first seal may be positioned in the chamber wall. In the second position, the first seal may seal between the inserter and the housing. In the second position, the activation tabs may be positioned outside the housing. In the second position, the cap may be attached to the inserter. In the second position, the cap may be detached from the inserter.

The funnel and a wetting mechanism may fit together with a push-fit. The wetting mechanism, funnel and sleeve may together define a cavity containing the catheter. The funnel and wetting mechanism may fit together to close the cavity with a push-fit.

Accordingly in an aspect of the invention there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, and wherein the funnel and wetting mechanism fit together to close the cavity with a push-fit.

Advantageously, the catheter assembly is thereby easier to use as a cavity can be formed around the catheter by simply push-fitting the funnel into the wetting mechanism. This can provide an effective barrier to unwanted leakage of liquid without requiring complicated manipulation of the funnel and wetting mechanism. In addition, as a wetting mechanism is provided, there is no requirement for the catheter assembly to provide a closed cavity prior to use, so the user does not need to manually separate the funnel and wetting mechanism prior to use and further the funnel and wetting mechanism do not need to provide a sterile-sealed cavity around the catheter, which again makes the catheter assembly easier to use and enables a simpler push-fit design.

The funnel may fit together with the wetting mechanism when the wetting mechanism is in the first position. The funnel may fit together with the wetting mechanism only when the wetting mechanism is in the first position. The funnel may be configured to move the inserter (or sealing member) into the first position. Thus, the wetting mechanism and funnel may be configured such that when push-fit together, (any further) release of wetting agent from the storage chamber is inhibited.

The wetting mechanism may comprise a bore sized to allow the catheter to pass out of the cavity. The bore may be provided by a cap end and/or an inserter tip of the wetting mechanism as described in more detail below.

The funnel and wetting mechanism may have corresponding shapes. The funnel and wetting mechanism may have corresponding sizes. An internal profile of one of the funnel or wetting mechanism may correspond to an external profile of the other of the funnel or wetting mechanism. An internal profile of the wetting mechanism may correspond to an external profile of the funnel to facilitate a push-fit. Preferably, the wetting mechanism may be configured to engage both an inside and an outside of the funnel when push-fit together, preferably simultaneously. The wetting mechanism may comprise one or more tabs configured to engage the funnel when push-fit with the wetting mechanism. The funnel may be configured to deform the one or more tabs when the funnel is push-fit with the funnel. The funnel may be configured to bend the one or more tabs when the funnel is push-fit with the funnel. The one or more tabs may be configured to engage the inside of the funnel. The one or more tabs may extend around only part of the funnel. The one or more tabs may comprise two or more tabs. The two or more tabs may be configured to engage opposing sides of the funnel. The one or more tabs may be provided by the activation tabs of the inserter. One or both of the funnel and wetting mechanism may be tapered. Thus, the funnel and wetting mechanism are sized to facilitate an effective push-fit. The tabs also assist locating of the funnel with the wetting mechanism to ensure an effective push-fit.

The wetting mechanism may be configured to receive the funnel. An internal profile of the wetting mechanism may correspond to an external profile of the funnel. The funnel may be tapered. An outlet end of the funnel may be configured to be inserted into the wetting mechanism. Thus, the wet end of the funnel is received by the wetting mechanism which helps ensure any leaks of liquid from the catheter assembly are minimised.

The funnel and wetting mechanism may have a different rigidity. One or both of the funnel and wetting mechanism may be resiliently deformable upon push-fitting of the funnel and wetting mechanism together. Preferably, the funnel is less rigid than the wetting mechanism. This helps to ensure the cavity is more tightly closed or even sealed by the funnel and wetting mechanism when push-fit together.

The wetting mechanism may comprise a cap. The cap may be configured to close the wetting mechanism before use. The cap may fit to the wetting mechanism with an interlocking mechanism. The interlocking mechanism may comprise cooperating apertures and tabs. The apertures may be provided on the cap. The tabs may be provided on an inserter tip of the wetting mechanism. Thus, the catheter is protected from damage until the point of use and the interlocking mechanism reduces the chance of inadvertent removal of the cap before use. The cap and interlocking mechanism are described further below.

The cap and funnel may be configured to fit to the same part of the wetting mechanism. The funnel may fit around the inserter, preferably when push-fit together with the wetting mechanism. The cap may fit around the inserter. Only one of the funnel and cap may be fit together with the wetting mechanism at a time. This ensures the catheter assembly is properly used as the user must remove the cap before they can then use the catheter or stow it by push-fitting the funnel with the wetting mechanism. It is also more intuitive as they funnel is push-fit into the same area that the cap came from.

The funnel may be configured to receive the inserter tip, preferably when the funnel is push-fit together with the wetting mechanism. Thus, the wet inserter/inserter tip of the wetting mechanism that has been inside the body is received by the funnel to help minimise leaks of liquid from the catheter assembly.

The catheter assembly may comprise an open configuration in which fluid flow through the funnel and out of the catheter assembly is permitted. When the catheter assembly is in the open configuration, the inserter may be in the first position or second position. The cap may be fitted into the wetting mechanism in the open configuration. When the cap is fitted into the wetting mechanism, the inserter may be in the first position. The inserter may only be moveable into the second position when the cap and/or funnel are not fitted into the wetting mechanism. This ensures release of wetting agent only when the user is ready to use the catheter and has removed the cap, as well as inhibiting further release after use.

The catheter assembly may comprise a closed configuration in which the cavity is closed to restrict fluid flow through the funnel and out of the catheter assembly. The funnel may be push-fit into the wetting mechanism in the closed configuration. When the catheter assembly is in the closed configuration, the inserter may be in the first position. The funnel and wetting mechanism may define a tortuous path for liquid between the funnel and the outside of the catheter assembly. The funnel and wetting mechanism may provide a liquid seal between the funnel and the outside of the catheter assembly. Thus, leaks of liquid from the catheter assembly after use are minimised giving the user more of an opportunity to stow or dispose of the catheter assembly without contacting liquid from inside the catheter.

The funnel may comprise a skirt. The skirt may be a part of the outlet portion. The skirt may be provided at the outlet end. The skirt may comprise a widened section of the outlet portion. The skirt may be about 0.5 mm, about 1mm or about 1.5mm wider than the rest of the outlet portion. The skirt may span at least 5%, at least 10%, or at least 15% of the length of the outlet portion. The skirt may span no more than 20%, no more than 15% or no more than 10% of the length of the outlet portion. The skirt preferably spans about 10% of the length of the outlet portion. The skirt may be configured to engage the wetting mechanism. Thus, the skirt provides a structure that can better engage the wetting mechanism.

The funnel may comprise a plurality of locating protrusions. The locating protrusions may be configured to engage the wetting mechanism. The locating protrusions may be configured to be overridden by the wetting mechanism to indicate when the funnel is push-fit together with the wetting mechanism. The locating protrusions are preferably provided in pairs. A pair of locating protrusions may be arranged adjacent to one another along the length of the funnel. A plurality of pairs of locating protrusions may be provided. The pairs of locating protrusions may be spaced around the circumference of the funnel. The pairs of locating protrusions may be evenly spaced around the circumference of the funnel. The pairs of locating protrusions may be arranged at the same point along the length of the funnel. The locating protrusions may be located approximately 10-30% along the length of the funnel from the outlet end.

Preferably there are at least four pairs of locating protrusions, for example four pairs, five pairs or most preferably six pairs of locating protrusions. The wetting mechanism may be configured to override one of each pair of the locating protrusions. The wetting mechanism may be configured to seat between the two locating protrusions in each pair of locating protrusions. Each locating protrusions may be any suitable shape. Preferably, each locating protrusion is hemispherical. Each locating protrusion may extend outward from the funnel. Each locating protrusion may extend outward from the funnel by at least 0.2 mm, at least 0.4 mm, at least 0.6 mm. Each locating protrusion may extend outward from the funnel by no more than 1 mm, no more than 0.8 mm, no more than 0.6 mm. Preferably each locating protrusion extends outward from the funnel by 0.6 mm. The locating protrusion may extend out past the external profile of the outlet end. The locating protrusion may extend out past the external profile of the skirt. Thus, the locating protrusions help control the push-fit engagement of the funnel and wetting mechanism.

The locator rib may be provided between the skirt and the inlet portion. The locator rib may be provided between the locating protrusions and the inlet portion.

The cap end (of the housing) may be configured to receive the funnel. The cap end may be annular. The cap end may be configured to provide a bore to allow the catheter to pass out of the wetting mechanism. An internal diameter of the cap end may correspond to the external profile of the funnel. An internal diameter of the cap end may be larger than the external diameter of the outlet end. An internal diameter of the cap end may be configured to override the locating protrusions. Thus, the cap end can seat onto the locating protrusions to locate the funnel in the wetting mechanism. The cap end thereby assists in preventing liquid leaks from the catheter assembly.

The activation tabs may extend outside an internal diameter of the skirt/outlet end. The skirt may be configured to engage the activation tabs. The activation tabs may be deformable by the skirt. Thus, the skirt may engage the inserter via the activation tabs.

The apertures and activation tabs thereby provide an interlocking mechanism to retain the cap on the wetting mechanism.

The cap end may prevent outward flexion of the cap while the inserter is in the first position.

The locator bumps may be substantially identical to the locating protrusions of the funnel. Thus, the funnel and cap are seated in the wetting mechanism in a very similar way.

The sleeve may be attached to the funnel. A fluid-tight seal may be provided between the sleeve and the funnel. A fluid path may be provided from the sleeve into the funnel. The fluid path may be directly from the sleeve into the funnel. Thus, liquid may efficiently pass from the catheter and/or sleeve into the funnel.

The sleeve may comprise a flexible plastics material. The sleeve may be liquid impermeable. The sleeve may comprise a thermoplastic polyurethane (TPU) or low-density polyethylene (LDPE). Thus, the sleeve is cheap and easy to produce and can be easily manipulated by the user during use.

The catheter may be an intermittent catheter. The catheter may be a urinary catheter. The catheter may be a male urinary catheter or a female urinary catheter. Preferably, the catheter is an intermittent male urinary catheter. Thus, the features of the present invention allow intermittent male urinary catheters to be more easily used which can be more difficult than for other types of catheter which are generally shorter.

The catheter may be formed of a material of the group comprising: polyvinyl chloride, polytetrafluoroethylene, polyolefins, latex, silicones, synthetic rubbers, polyurethanes, polyesters, polyacrylates, polyamides, thermoplastic elastomeric materials, styrene block copolymers, polyether block amide, thermoplastic vulcanizates, thermoplastic copolyesters, thermoplastic polyamides, and water disintegrable or enzymatically hydrolysable material, or combinations, blends or copolymers of any of the above materials.

Preferably, the catheter is formed of a polyolefin material, in particular polyethylene and/or polypropylene.

Preferably, the catheter is formed of a thermoplastic elastomeric material.

The sleeve may be configured to enclose
at least 90% of the length of the catheter. The sleeve and funnel may together enclose a majority of, or at least 90% of the length of the catheter. The wetting mechanism, sleeve and funnel may together enclose the entire length of the catheter.

The catheter assembly may be provided in a pouch. The pouch may be sealed. The pouch may provide a sterile seal or may be a "sterile-sealed" pouch. Thus, aspects of the invention provide for a packaged catheter assembly comprising a sterile-sealed pouch containing a catheter assembly as described herein, and preferably a catheter assembly of the second aspect. Thus, there is provided a packaged catheter assembly comprising a sterile-sealed pouch containing a catheter assembly, the catheter assembly a catheter comprising a proximal end for insertion into the body and a distal end, and a funnel configured to direct fluid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth.

Thus, there is also provided a packaged catheter assembly comprising a sterile-sealed pouch containing a catheter assembly, the catheter assembly comprising a catheter comprising a proximal end for insertion into the body and a distal end, and a sleeve configured to enclose the catheter from the proximal end to the distal end, wherein the catheter assembly comprises a funnel configured to direct liquid out of the distal end of the catheter and the funnel comprises a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations.

Thus, there is also provided a packaged catheter assembly comprising a sterile-sealed pouch containing a catheter assembly, the catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, and wherein the funnel and wetting mechanism fit together to close the cavity with a push-fit.

The catheter assembly is preferably provided in the sterile-sealed pouch in the open configuration. The cap is preferably attached to the wetting mechanism inside the sterile-sealed pouch. Thus the catheter is more easy to use as the user does not need to open the end of the funnel before use.

The pouch may comprise a flexible plastics material. The pouch may be liquid impermeable. The pouch may comprise any one or more of: polypropylene (PP), polyethylene terephthalate (PET), low density polyethylene (LDPE), polyethylene terephthalate polyester (MET PET) or orientated polypropylene (OPP), for example. Thus, the pouch is cheap and easy to produce and can be easily manipulated by the user during use.

The pouch may be formed of two walls. The two walls may be joined at their periphery by a peripheral seal. The two walls may be formed of a flexible plastics material, for example one as described in relation to the pouch above. The peripheral seal may comprise any one or more of: a weld; mechanical seal; heat seal; pressure seal; adhesive; solvent bond; ultraviolet bond; ultrasonic weld; laser weld; impulse weld; or friction weld.

The pouch may comprise an interaction region defining a first point of contact for a user of the packaged catheter assembly. The interaction region may facilitate user access to the contents of the pouch. Actuation of the interaction region may open or form an opening in the pouch. The interaction region may comprise a tear-away region of the pouch. The pouch can thus be easily accessed by the user.

Many variations on the features of the funnel and catheter assembly as described are envisaged and any one or more of the features of them can be combined in many different ways to provide different advantages, without necessarily requiring all the features of the first/second aspects as outlined above. Certain specific advantageous embodiments of the funnel and catheter assembly as described herein are set out below by way of example.

In one example, there is provided a catheter assembly comprising a catheter comprising a proximal end for insertion into the body and a distal end, and a funnel configured to direct fluid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel.

In one example, there is provided a funnel for a catheter, the funnel being configured to direct fluid out of a distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the external profile defined by the tips changes along the length of the funnel.

There is also provided a catheter assembly comprising a catheter comprising a proximal end for insertion into the body and a distal end, and a funnel configured to direct fluid out of a distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the external profile defined by the tips changes along the length of the funnel.

The changing external profile defined by the tips thereby allows for the design of an external profile that different from the internal profile and more suited to a specific task such as being more comfortable or easy to manipulate for users with limited dexterity.

In another example there is provided a funnel for a catheter, the funnel being configured to direct fluid out of a distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the projections have a radial length defined as the distance the projections extend from the internal tube in a radial direction and the radial length of the projections changes along the length of the funnel.

There is also provided a catheter assembly comprising a catheter comprising a proximal end for insertion into the body and a distal end, and a funnel configured to direct fluid out of a distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the projections have a radial length defined as the distance the projections extend from the internal tube in a radial direction and the radial length of the projections changes along the length of the funnel.

In another example, there is provided a funnel for a catheter, the funnel being configured to direct fluid out of a distal end of the catheter, wherein the funnel comprises a tube defining an internal profile of the funnel and the tube comprises two or more catheter receiving zones, wherein each catheter receiving zone is configured to receive and retain a different type and/or size of catheter and the two or more catheter receiving zones are arranged in series along the length of the funnel.

There is also provided a catheter assembly comprising a catheter comprising a proximal end for insertion into the body and a distal end, and a funnel configured to direct fluid out of a distal end of the catheter, wherein the funnel comprises a tube defining an internal profile of the funnel and the tube comprises two or more catheter receiving zones, wherein each catheter receiving zone is configured to receive and retain a different type and/or size of catheter and the two or more catheter receiving zones are arranged in series along the length of the funnel.

Thus, the same funnel can be used for two or more different sizes/types of catheter whereas existing funnels are specific to only one size/type of catheter. This reduces manufacturing complexity and cost as less types/sizes of funnel are required. In addition, as the two zones are provided in series, then the fitting process is essentially the same for whatever catheter is used with the funnel as they can all be fitted into the same hole.

In another example, there is provided a catheter assembly comprising an intermittent urinary catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, preferably wherein the catheter is an intermittent male urinary catheter. The features of the funnel are particularly suited for use with an intermittent urinary catheter as they provide a better handling experience for the user.

In another example, there is provided a catheter assembly comprising a catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the stepped region comprises at least two steps in the internal profile. Thus, more complex internal structure can be realised without impacting the external profile.

In another example, there is provided a catheter assembly comprising a catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the tube has a substantially constant wall thickness. Thus, the funnel is easier to manufacture effectively and without artefacts, such as by injection moulding.

In another example, there is provided a catheter assembly comprising a catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the internal profile comprises a step at a transition point along the length of the funnel and the projections are provided on both sides of the transition point. Thus, the projections effectively "hide" the transition point and provide a smooth outside profile for the funnel.

In another example, there is provided a catheter assembly comprising a catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the projections have a radial length defined as the distance the projections extend from the internal tube in a radial direction and the radial length changes along the length of the funnel. Thus, changes in the internal profile can be easily accommodated with changes in length of the projections.

In another example, there is provided a catheter assembly comprising a catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the tube comprises two or more catheter receiving zones, wherein each catheter receiving zone is configured to receive a different type and/or size of catheter. Thus, the funnel can fit onto two different types of catheter, this reduces manufacturing cost/complexity when making multiple assemblies for two different catheters.

In another example, there is provided a catheter assembly comprising a catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the tube comprises two or more catheter receiving zones arranged in series along the length of the funnel, wherein each catheter receiving zone is configured to receive a different type and/or size of catheter. Thus, as the zones are in series, they enable different catheters to simply be inserted into the same end of the funnel and fit into place easily.

In another example, there is provided a catheter assembly comprising a catheter comprising a proximal end for insertion into the body and a distal end, and a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the plurality of projections comprise a plurality of ribs and the plurality of ribs comprise one or more axial ribs extending along the length of the funnel. This can help provide additional friction to the user to allow easier rotation of the funnel about its axis.

In another example there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, wherein the funnel and wetting mechanism fit together to close the cavity with a push-fit, wherein the funnel is less rigid than the wetting mechanism. This is advantageous as the wetting mechanism should not easily bend or flex as this may result in premature release of wetting agent.

In another example there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, wherein the funnel and wetting mechanism fit together to close the cavity with a push-fit, wherein the proximal end of the catheter is provided in the sleeve. Thus, the proximal end of the catheter may be outside the wetting mechanism. This facilitates more effective wetting of the catheter if wetting agent is released into the sleeve.

In another example there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, wherein the funnel and wetting mechanism fit together to close the cavity with a push-fit, wherein the wetting mechanism is configured to be user actuatable to independently release the wetting agent to wet the catheter. Thus, the user can independently select when to wet the catheter which can avoid inadvertent release of wetting agent, such as where the movement of the catheter causes release of wetting agent.

In another example there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, wherein the funnel and wetting mechanism fit together to close the cavity with a push-fit, wherein the wetting mechanism and funnel are configured such that when push-fit together release of wetting agent from the storage chamber is inhibited. Thus, flow of fluid out of the wetting mechanism is restricted reducing leaks.

In another example there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, wherein the funnel and wetting mechanism fit together to close the cavity with a push-fit, wherein the wetting mechanism is configured to simultaneously engage both an inside and an outside of the funnel when push-fit together. Thus, the push-fit seal involves engagement of both the inside and outside of the funnel which makes it more secure.

In another example there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, wherein the funnel and wetting mechanism fit together to close the cavity with a push-fit, wherein the wetting mechanism comprises an inserter, and the funnel is configured to fit around the inserter. In a preferred embodiment, the inserter comprises an inserter tip and the funnel is configured to receive the inserter tip when the funnel is push-fit together with the wetting mechanism. Thus, the funnel may receive the inserter (or inserter tip) that is likely to be wet after use, this better contains any residual liquid and reduces hygienic risks.

In another example there is provided a catheter assembly comprising a catheter comprising a proximal end for insertion into the body and a distal end, a wetting mechanism, a funnel, and a tubular sleeve, wherein the wetting mechanism and funnel are provided at either end of the sleeve and the wetting mechanism, funnel and sleeve together define a cavity containing the catheter, wherein the wetting mechanism comprises a storage chamber configured to retain a wetting agent, the wetting mechanism is configured to release the wetting agent to wet the catheter in use, wherein the funnel and wetting mechanism fit together to close the cavity with a push-fit, wherein the wetting mechanism comprises one or more tabs configured to engage the funnel when push-fit with the wetting mechanism. Thus, the tabs help locate the funnel with respect to the wetting mechanism to provide a more effective push-fit.

In another example there is provided a catheter assembly comprising an intermittent urinary catheter comprising a proximal end for insertion into the body and a distal end, and a funnel configured to direct liquid out of the distal end of the catheter, the funnel comprising a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations. The features of the funnel are particularly helpful when used with an intermittent urinary catheter as the user must operate, and dispose of, the catheter typically alone and in public places, so the provision of the closure element helps avoid spillages and other hygienic issues before disposal.

In another example there is provided a catheter assembly comprising a catheter comprising a proximal end for insertion into the body and a distal end, and a funnel configured to direct liquid out of the distal end of the catheter, the funnel comprising a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations, wherein the closure element comprises a retainer configured to retain the closure element in the stowed position, preferably wherein the retainer is breakable to release the closure element from the stowed position. This helps ensure the closure element does not interfere with use of the catheter and fluid flowing out of the funnel until it is needed to close the funnel.

In another example there is provided a catheter assembly comprising a catheter comprising a proximal end for insertion into the body and a distal end, and a funnel configured to direct liquid out of the distal end of the catheter, the funnel comprising a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations, wherein the closure element comprises a plug which plugs the funnel in the closed configuration. This enables more efficient sealing of the funnel and reduces the size of the catheter assembly as the closure element can fit within the funnel.

In another example there is provided a catheter assembly comprising a catheter comprising a proximal end for insertion into the body and a distal end, and a funnel configured to direct liquid out of the distal end of the catheter, the funnel comprising a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations, wherein in a stowed position, the catheter assembly is in the open configuration and the closure element does not impede liquid flow out of the funnel, and wherein the closure element comprises a connector attached to the funnel and a closure body configured to inhibit liquid flow out of the funnel in the closed configuration, wherein the connector is substantially S-shaped in the stowed position. This allows the closure body to be efficiently stowed next to the funnel prior to use.

In another example there is provided a catheter assembly comprising a catheter comprising a proximal end for insertion into the body and a distal end, and a funnel configured to direct liquid out of the distal end of the catheter, the funnel comprising a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations, wherein the catheter is rotation dependent and comprises an insertion orientation configured to enable the catheter to release fluid from the body, wherein the closure element is arranged above or to one side of the funnel when the catheter is in the insertion orientation. This helps ensure that the closure element does not inadvertently contact liquid flowing through the catheter in use.

In another example there is provided a catheter assembly comprising a catheter comprising a proximal end for insertion into the body and a distal end, and a funnel configured to direct liquid out of the distal end of the catheter, the funnel comprising a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations, wherein the closure element and/or funnel comprises one or more sealing protrusions configured to provide a seal between the funnel and closure element. Thus, the seal between the funnel and closure element may be enhanced with sealing protrusions.

Of course, the funnel and catheter assembly examples above may also be combined with any one or more of the features of a funnel or catheter assembly as described herein. For example, there may be provided a funnel for a catheter, the funnel being configured to direct fluid out of a distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth, wherein the funnel comprises a closure element configured to inhibit fluid flow through the catheter and integrally formed with the funnel.

The invention also relates to a method of manufacturing a catheter assembly.

According to a third aspect of the present invention there is provided a method of manufacturing a catheter assembly comprising providing a catheter comprising a proximal end for insertion into the body and a distal end, and forming a funnel configured to direct liquid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the internal profile comprises a stepped region and a corresponding region of the external profile is smooth.

The funnel may be formed by injection moulding.

In another example, there is provided a method of manufacturing a catheter assembly comprising providing a catheter comprising a proximal end for insertion into the body and a distal end, and forming a funnel configured to direct liquid out of the distal end of the catheter, and attaching the funnel to the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, wherein the external profile defined by the tips changes along the length of the funnel.

Preferably, the plurality of protrusions are formed by injection moulding. Preferably, the protrusions are integrally formed with the funnel.

In another example, there is provided a method of manufacturing a catheter assembly comprising forming a funnel configured to direct liquid out of the distal end of the catheter, wherein the funnel comprises a tube defining an internal profile of the funnel wherein the method includes the step of shaping the tube so as to define two or more catheter receiving zones provided in series along the tube, wherein each catheter receiving zone is configured to receive and retain a different type and/or size of catheter, the method further comprising selecting a catheter that corresponds to one or the catheter receiving zones and attaching the funnel to the distal end of the catheter.

In another example, there is provided a method of manufacturing two or more catheter assemblies, the method comprising forming a funnel configured to direct liquid out of the distal end of the catheter, wherein the funnel comprises a tube defining an internal profile of the funnel wherein the method includes the step of shaping the tube so as to define two or more catheter receiving zones provided in series along the tube, wherein each catheter receiving zone is configured to receive and retain a different type and/or size of catheter, repeating the step of forming a funnel to leave two or more identical funnels, then selecting two or more different sizes and/or types of catheter, wherein each size and/or type of catheter corresponds to one of the two or more catheter receiving zones, and attaching each funnel to the distal end of one of the catheters to leave two or more catheter assemblies.

In another example there is provided a method of manufacturing a catheter assembly comprising, providing a catheter comprising a proximal end for insertion into the body and a distal end, and forming a funnel configured to direct liquid out of the distal end of the catheter, and attaching the funnel to the distal end of the catheter, wherein the funnel comprises a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations.

The funnel and closure element may be formed simultaneously. The closure element may be formed by injection moulding. The funnel and closure element may be formed by injection moulding.

In another example there is provided a method of manufacturing a catheter assembly, the method comprising providing a catheter, a tubular sleeve, a wetting mechanism and a funnel, attaching the wetting mechanism and funnel to either end of the sleeve, and inserting the catheter into the sleeve such that a cavity is formed around the catheter by the wetting mechanism, sleeve and funnel, and the funnel is configured to direct liquid out of the distal end of the catheter, wherein the wetting mechanism comprises a storage chamber configured to retain a wetting agent, the wetting mechanism is configured to release the wetting agent to wet the catheter in use, and wherein the funnel and wetting mechanism are constructed to fit together to close the cavity with a push-fit.

The method may comprise providing the catheter assembly to the user with the cavity open, for example the funnel and wetting mechanism not push-fit together. The method may comprise fitting a cap to the wetting mechanism.

According to a fourth aspect of the invention there is provided a method of handling a catheter assembly, the catheter assembly comprising a catheter comprising a proximal end for insertion into the body and a distal end, and a funnel configured to direct fluid out of the distal end of the catheter, wherein the funnel comprises a tube defining an internal profile of the funnel and a plurality of projections extending outward from the tube, wherein each projection comprises a tip distal from the tube and the tips of the projections together define at least part of an external profile of the funnel, the method comprising grasping the projections so as to control movement of the catheter.

The method may comprise introducing the catheter by its proximal end into the urethra. The method may comprise allowing fluid to pass out of the body via the catheter. Consequently, the method may be a method of using a packaged catheter assembly.

In one example, there is provided a method of stowing a catheter assembly after use, the catheter assembly comprising a catheter comprising a proximal end for insertion into the body and a distal end, and a funnel configured to direct liquid out of the distal end of the catheter, the method comprising moving a closure element of the funnel from an open configuration in which liquid flow out of the funnel is permitted by the closure element to a closed configuration in which the closure element inhibits liquid flow out of the funnel., wherein the funnel and closure element are connected in both the open and closed configurations.

In another example, there is provided a method of stowing a catheter assembly after use, the catheter assembly comprising a catheter comprising a proximal end for insertion into the body and a distal end, a tubular sleeve, a wetting mechanism, and a funnel configured to direct liquid out of the distal end of the catheter, wherein the wetting mechanism and funnel are provided at either end of the sleeve, wherein the wetting mechanism comprises a storage chamber configured to retain a wetting agent, the wetting mechanism is configured to release the wetting agent to wet the catheter in use, and the method comprises push fitting the funnel and wetting mechanism together to form a closed cavity around the catheter with the sleeve.

The funnel and catheter assemblies described in relation to the methods of the third and fourth aspects, as well as the associated different additional examples provided, may be the funnel according to the first aspect above, and/or the catheter assembly according to the second aspect above respectively. Each may of course include any one or more features optional or otherwise as described in relation to the first and second aspects and the associated different aspects and examples of funnels and catheter assemblies as described herein.

The methods of the third and fourth aspect, as well as the associated examples, may also include any one or more features, optional or otherwise, of one another as well the first and second aspects and associated other aspects, examples and embodiments described above.

### Detailed Description of the Invention

In order that the invention may be more clearly understood one or more embodiments thereof will now be described, by way of example only, with reference to the accompanying drawings, of which:
- Figure 1: is a first embodiment of a packaged catheter assembly with the catheter assembly in an open configuration and with a closure element in a stowed position;
- Figure 2: is the funnel of the catheter assembly of Figure 1 in the open configuration and with the closure element not in the stowed position;
- Figure 3: is the funnel of the catheter assembly of Figure 1 in a closed configuration;
- Figure 4: is a perspective view of just the funnel of the catheter assembly of Figure 1 in the open configuration with the closure element in a stowed position;
- Figure 5: is a cross-sectional view of the funnel of the catheter assembly of Figure 1 in the open configuration and showing funnel ribbing;
- Figure 6: is a cross-sectional view of just the funnel of the catheter assembly of Figure 1 in the open configuration showing the closure element in a stowed position;
- Figure 7: is a cross-sectional view of just the funnel of the catheter assembly of Figure 1 in the closed configuration;
- Figure 8: is the wetting mechanism of the catheter assembly of Figure 1 before wetting the catheter;
- Figure 9: is a cross sectional view of the wetting mechanism of the catheter assembly of Figure 1 before wetting the catheter;
- Figure 10: is a cross sectional view of the wetting mechanism of the catheter assembly of Figure 1 during wetting the catheter;
- Figure 11: is a cross sectional view of the wetting mechanism of the catheter assembly of Figure 1 with the catheter extending out of the wetting mechanism;
- Figure 12: is a second embodiment of a packaged catheter assembly;
- Figure 13: is a cross-sectional view of the funnel of the catheter assembly of Figure 12;
- Figure 14: is the funnel of the catheter assembly of Figure 12;
- Figure 15: is a cross sectional view of the wetting mechanism of the catheter assembly of Figure 12 with the funnel push-fit together with the wetting mechanism; and
- Figure 16: is the catheter assembly of Figure 12 with the funnel push-fit together with the wetting mechanism and re-packaged into the pouch after use.

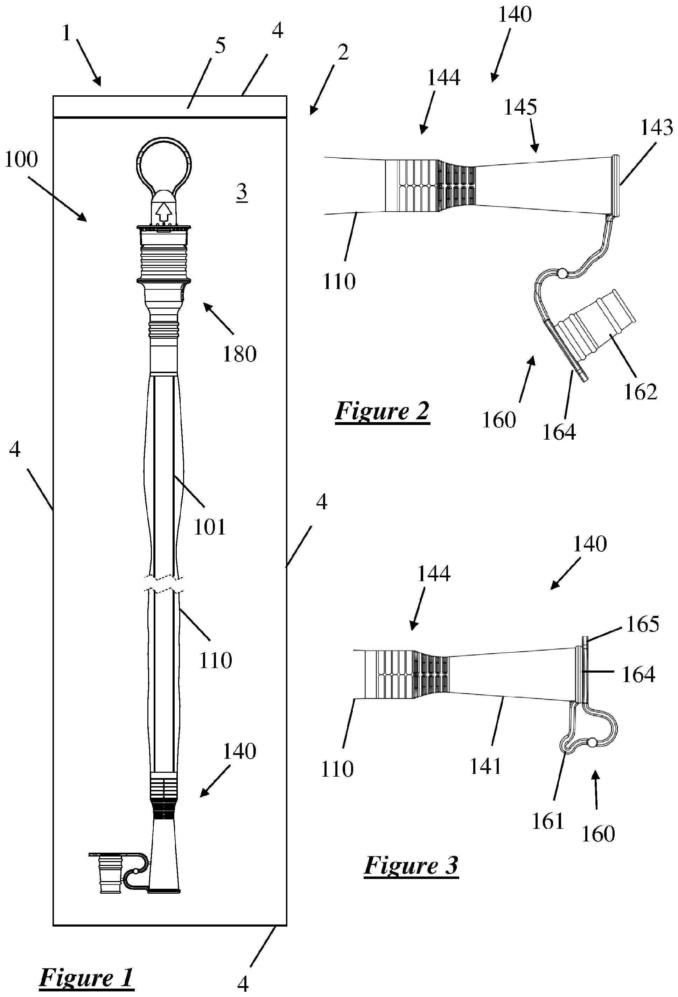

Referring to Figures 1 to 11, a first embodiment of packaged catheter assembly 1 comprises a pouch 2 and a catheter assembly 100.

In this embodiment, the pouch 2 provides a sterile package for the catheter assembly 100 to ensure it remains sterile prior to use, the packaged catheter assembly 1 is therefore a sterile-sealed packaged catheter assembly. The pouch 2 comprises two walls 3 and a peripheral seal 4 joining them at their periphery. The walls 3 are constructed from a flexible plastics material and are liquid impermeable to maintain the sterility of the contents. The walls 3 in this embodiment are constructed from any one or more of: polypropylene (PP), polyethylene terephthalate (PET), low density polyethylene (LDPE), polyethylene terephthalate polyester (MET PET) or orientated polypropylene (OPP).

The peripheral seal 4 is formed by any suitable way of sealing the walls 3 together to form a sterile seal, for example the peripheral seal 4 may comprise any one or more of: a weld; mechanical seal; heat seal; pressure seal; adhesive; solvent bond; ultraviolet bond; ultrasonic weld; laser weld; impulse weld; or friction weld.

In this embodiment, the pouch 2 comprises an interaction region in the form of a tear-away region 5. The tear-away region 5 spans across one side of the pouch 2 along the peripheral seal 4. The tear-away region 5 provides a first point of contact for a user of the packaged catheter assembly 1 and is configured to facilitate user access to the contents of the pouch 2. In this embodiment, the tear-away region 5 may be torn to form an opening in the pouch 2 as described further below.

In this embodiment, the catheter assembly 100 comprises a catheter 101, a sleeve 110, a funnel 140 and a wetting mechanism 180. The catheter 101 is an intermittent male urinary catheter. The catheter 101 comprises a proximal end 102 for insertion into the body and a distal end 103. In this embodiment, the catheter 101 comprises a surface which is configured to be activated by contact with a wetting agent. Once activated the catheter 101 is lubricious and ready to be inserted into the body. This is described in more detail below.

In this embodiment, the sleeve 110 comprises a liquid impermeable flexible plastics material, for example a thermoplastic polyurethane (TPU) or low-density polyethylene (LDPE). The sleeve 110 is configured to enclose the catheter along the majority of, if not all of, its length between the proximal 102 and distal 103 ends. The sleeve 110 is tubular and is coupled at one end to the funnel 140 and at the other end to the wetting mechanism 180. The catheter 101 is positioned within the sleeve 110 and is positioned completely within a cavity formed by the sleeve 110, funnel 140 and wetting mechanism 180. The catheter 101 is thereby protected even after the pouch 2 is opened and the sleeve 110 enables the user to more easily handle and use the catheter 101 without compromising its sterility.

In this embodiment, the funnel 140 is coupled to the distal end 103 of the catheter 101 and the sleeve 110. The funnel 140 is relatively rigid compared to the catheter 101 and sleeve 110 and in this embodiment comprises a flexible plastics material, for example linear low-density polyethylene (LLHDPE). The funnel 140 is configured to receive liquid from the distal end 103 of the catheter 101 and then direct the flow of liquid out of the catheter assembly 100.

In this embodiment, the funnel 140 is generally tubular and is formed by a tube 141 extending between an inlet end 142 and an outlet end 143. The funnel 140 comprises an inlet portion 144 extending from the inlet end 142 and an outlet portion 145 extending from the outlet end 143, the inlet portion 144 and the outlet portion 145 meeting at the narrowest point along the tube 141. The inlet portion 144 is configured to receive the distal end 103 of the catheter 101 and provide a liquid path from inside the catheter 101 into the funnel 140. The outlet portion 145 is configured to direct the flow of liquid out of the funnel 140.

In this embodiment, the outlet portion 145 is longer than the inlet portion 144, and the outlet portion 145 covers about 60-70% of the length of the funnel 140.

In this embodiment, the tube 141 has a substantially constant wall thickness. In the outlet portion 145, the tube 141 defines both the internal and external profile of the funnel 140 and is tapered with a frustoconical shape that diverges from the inlet portion 144 towards the outlet end 143. The outlet portion 145 is widest at the outlet end 143 with an external diameter of about 12 mm and an internal diameter of about 8-9 mm.

On the outside of the outlet portion 145, there is provided a locator in the form of a locator rib 146 which extends axially along the length of the funnel 140 and outlet portion 145. The locator rib 146 is positioned centrally with respect to the length of the outlet portion 145 and is configured to align with a curve in the catheter 101 so that the user can correctly position it within the body, for example, when using a rotation dependent catheter such as a coudé catheter. Such catheters may have an insertion orientation that enables them to release fluid from the body.

The funnel 140 also comprises a closure element 160 which is attached to the outlet end 143 and is described in more detail below.

In this embodiment, where the inlet portion 144 meets the outlet portion 145, the tube 141 is about 3-5 mm wider on the outlet portion 145 side. Consequently, a flange 147 is provided to connect the inlet portion 144 to the outlet portion 145 to facilitate the step-change in the internal profile of the funnel 140.

In this embodiment, on the outlet portion 145 side of the flange 147 and on the inside of the funnel 140, a sealing ring 148 is provided which extends from the flange 147 towards the outlet end 143. The sealing ring 148 facilitates sealing of the funnel 140 to an external connector device (not shown), for example to allow liquid to pass from the funnel 140 into the external connector device and be collected for analysis or testing. Of course, generally the funnel 140 will be used without the external connector device.

In this embodiment, the funnel 140 is configured to receive and retain more than one type and/or size of catheter. In particular, the funnel 140 is configured to be able to receive and retain two different sizes of catheter 101. The funnel 140 thereby comprises two catheter receiving zones (CRZ): a first CRZ 149 and a second CRZ 150. Each CRZ is configured to receive and retain a certain type/size of catheter.

In this embodiment, the internal profile of the inlet portion 144 is defined by the tube 141 and changes in the internal profile along the length of the inlet portion 144/funnel 140 provide the first and second CRZs 149, 150. The two CRZs are provided in series and coaxially with the tube 141 extending from the inlet end 142 to the first CRZ 149, then the second CRZ 150 and finally to the outlet portion 145.

In this embodiment, the internal profile of the funnel 140 narrows from the inlet end 142 towards the outlet portion 145. The internal profile tapers down from 4-8 mm at the inlet end 142 by about 1 mm to the first CRZ 149. The internal profile is then constant across the first CRZ 149, then steps down to the second CRZ 150, which has a diameter about 1 mm less than the first CRZ 149. The internal profile is then constant across the second CRZ 150 and then steps down again before meeting the outlet portion 145.

As described above, at the end of each CRZ distal from the inlet end 142, a catheter stop is provided in the form of a catheter flange 151. The catheter flange 151 provides a step in the internal profile of the funnel 140 and helps prevent over insertion of a catheter into a respective CRZ 149, 150. The inlet portion 144 therefore comprises a stepped region of the internal profile of the funnel 140.

In this embodiment, the catheter 101 is sized to fit into the first CRZ 149 and therefore, the distal end 103 of the catheter 101 is received into the first CRZ 149 such that liquid can pass from the catheter 101, through the distal end 103 and into the funnel 140 as described further below.

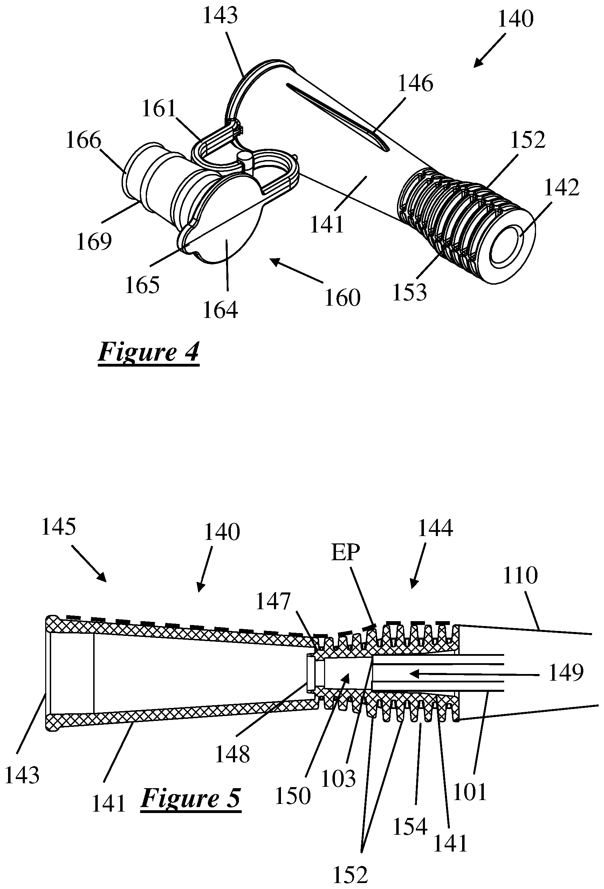

In this embodiment, the funnel 140 comprises a plurality of protrusions in the form of circumferential ribs 152 and axial ribs 153. The ribs 152, 153 each extend outward from the tube 141 and comprise a tip distal from the tube 141, the tips of the ribs 152, 153 together define the external profile of the funnel 140 for substantially all of the inlet portion 144, as indicated by the broken line EP in Figure 5. This allows the external profile of the funnel 140 to be different from its internal profile, which is restricted due to the need to provide the first and second CRZ 149, 150. In addition, gaps 154 are provided between adjacent ribs 152, 153. This allows the funnel 140 to be constructed with less material than a filled design, while also avoiding sink mark artefacts that can occur in injection moulded items of varying thickness. The ribs 153, 153 and gaps 154 also provide a more easily gripped external surface, making the funnel easier to handle.

In this embodiment, the ribs 152, 153 are tapered slightly with the tips being the narrowest part of each rib 152, 153. This makes them easier to manufacture by injection moulding. In addition, the tip of each rib 152, 153 is substantially planar, this helps them to define a smoother and more comfortable to hold external profile for the funnel 140 without sharp edges.

In this embodiment, eleven circumferential ribs 152 are provided, starting with one at the inlet end 142 and then being evenly spaced across the inlet portion 144 to the flange 147 which connects to the outlet portion 145. The protrusions are thereby only provided on one side of a transition point in the internal profile defined where the outlet and inlet portions meet. The protrusions are also provided across a transition point defined between the first and second CRZ 149, 150, but have different lengths across this transition point. The circumferential rib 153 provided at the inlet end 142 thereby provides an inlet flange 155 to form the inlet end 142. The inlet flange 155 having an external diameter of about 10 mm in this embodiment.

In this embodiment, four axial ribs 153 are provided which extend from the inlet flange 155 to the outlet portion 145. The axial ribs 153 therefore also define the external profile of the funnel 144 along the inlet portion 144. Each axial rib 153 intersects all of the circumferential ribs 152, this helps the ribs 152, 153 to support one another and prevent damage to them during use. The four axial ribs 153 are spaced evenly around the circumference of the funnel 140.

In this embodiment, the external profile of the inlet portion 144 is shaped to provide a premium look through smoothly changing features that avoid unnecessary step changes in size. The external profile is therefore smooth across the stepped region of the internal profile in the inlet portion 144.

The external profile of the inlet portion 144 is shaped to match the external profile of the outlet portion 145 where the two meet. Consequently, the ribs 152, 153 have a radial length, measured as the radial distance from the tip of the rib 152, 153 to the tube 141, that is substantially the same as the size of the flange 147 adjacent to the flange 147. Thus, the external profile (see broken line EP in Figure 5) is smooth where the inlet and outlet portions 144, 145 meet. The length of the ribs 152, 153 then increases towards the inlet end 142 such that the external profile curves outward with concave curvature from the outlet portion 145 towards the inlet end 142. Over this curved region means that the external profile is diverging which provides a shape that is more easily gripped by the user and is better suited to applying a force along the axis of the catheter 101 for insertion/removal of the catheter from the body.

In this embodiment, the curved region spans about half the length of the inlet portion, for example 7 mm, and constitutes an increase in the diameter of the external profile of the catheter from about 8 mm to 10 mm. The ribs 152, 153 are then shaped to provide a constant external profile over the remainder of the inlet portion 144 to the inlet end 142. The ribs 152, 153 therefore decrease in radial length slightly as they approach the inlet end 142 due to the tapering of the tube 141 which is largest at the inlet end 142. This constant region can be more suited to engaging the sleeve 110, such as where it is fitted over a part of the funnel 140.

As mentioned above, in this embodiment the funnel 140 comprises a closure element 160 configured to inhibit the flow of liquid out of the funnel 140. To allow control of flow out of the funnel 140, the closure element 160 is moveable between an open configuration in which liquid flow out of the funnel 140 is permitted and a closed configuration in which liquid flow out of the funnel 140 is inhibited.

In this embodiment, the closure element 160 comprises a connector 161 to attach the closure element 160 to the funnel 140 and a closure body in the form of a plug 162 configured to inhibit liquid flow out of the funnel 140 in the closed configuration by plugging the outlet end 143. In this embodiment, the connector 161 is elongate and is attached at one end to the funnel 140 adjacent the outlet end 143. The connector 161 extends in a radial direction, perpendicular to the length of the funnel 140, from the funnel 140 and then connects to the plug 162 at the other end. The connector 161 is flexible and thereby allows movement and rotation of the plug 162 with respect to the funnel 140 so that it can adopt the open and closed configurations as described further below.

In this embodiment, the plug 162 is tubular and shaped to match the internal profile of the funnel 140 so that it can be push-fit into the outlet end 143. The plug 162 is therefore generally cylindrical but with an open insertion end 163 that is configured to be inserted into the outlet end 143 first. The plug 162 is tapered down towards the insertion end 163.

In this embodiment, the plug 162 comprises a cap end 164 distal from the insertion end 163. The plug 162 has a length defined between the insertion and cap ends 163, 164 of about 40% the length of the funnel 140. The cap end 164 is formed by a cap that covers the end of the plug 162 with an outer diameter that matches the external profile of the outlet end 143. The cap end 164 thereby provides a stop that prevents over insertion of the plug 162 into the funnel 140 by bearing against the outlet end 143.

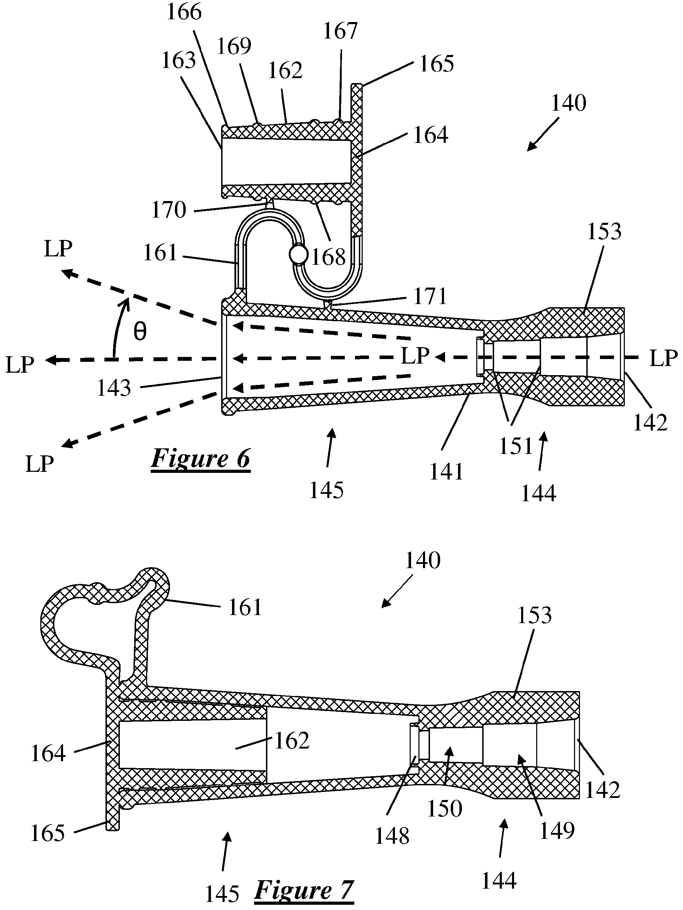

In this embodiment, the connector 161 attaches to one side of the cap end 164 and extends away from the plug 162 perpendicular to the length of the plug 162. Thus, when in the closed configuration, the connector 161 extends from the plug 162 and funnel 140 in substantially the same plane, or direction if rotationally aligned and as shown in Figure 7.

In this embodiment, the cap end 164 comprises a release mechanism in the form of a tab 165 that extends out from the cap end in a direction perpendicular to the length of the plug 162 and on an opposite side from where the connector 161 attaches to the cap end 164. Thus, the tab 165 extends outside the external profile of the funnel 140 when in the closed configuration and allows the user to more easily remove the plug 162 from the funnel 140 if desired.

In this embodiment, the plug 162 comprises four sealing protrusions in the form of sealing ribs which each extend around the circumference of the plug 162. The sealing ribs are resiliently deformable (along with the rest of the plug 162) and extend from the surface of the plug 162 to provide a more effective seal between the plug 162 and funnel 140 when in the closed configuration.

In this embodiment, the sealing ribs comprise a tip rib 166 arranged around the insertion end 163 of the plug 162. The sealing ribs also comprise a cap rib 167 provided adjacent to the cap end 164 on the plug 162, in this embodiment, the cap rib 167 is spaced from the cap end 164 by 10-20% of the length of the plug 162. The sealing ribs also comprise first and second intermediate ribs 168, 169 provided between the tip rib 166 and cap rib 167. The first intermediate rib 168 is provided towards the insertion end 163 approximately 20-30% of the length of the plug 162 from the insertion end 163. The second intermediate rib 169 is provided towards the cap end 164 approximately 20-40% of the length of the plug 162 from the cap end 164. Thus, the sealing ribs help ensure a better seal between the plug 162 and funnel 140.

In this embodiment, the closure element 160 comprises a retainer in the form of first and second retention links 170, 171. The retention links 170, 171 are configured to retain the closure element in a stowed position in which it does not impede liquid flow out of the funnel 140. That is, in the stowed position, the closure element 160 is positioned such that it is completely outside a liquid flow path (see LP denoted by broken arrows in Figure 6) through and out of the funnel 140. Thus, the closure element 160 does not get wet during use which makes it easier and more hygienic to handle and use. Consequently, as described above, in the open configuration the closure element 160 might be in the stowed position in which it doesn't impede liquid flow out of the funnel 140 or it might just be in a position where it permits liquid flow out of the funnel but does still impede the flow outside the funnel 140, for example it might be positioned in the liquid flow path outside the funnel 140 but will still allow liquid to flow out of the funnel. Whereas in the closed configuration, the plug 162 inhibits liquid flow out of the funnel 140. That is, the plug 162 is in a position which prevents flow of liquid out of the funnel in normal use.

In this embodiment, the liquid path through the funnel is defined by the internal profile of the funnel 140. As shown in Figure 6, the liquid path substantially fills the funnel 140 with liquid flowing through the funnel 140 from the inlet end 142 to the outlet end 143 and generally along an axis parallel to the length of the funnel 140. Once the liquid exits the funnel 140 at the outlet end 143, the liquid path diverges from its path through the funnel in a conical shape. The conical shape being defined by an aperture half angle, θ. In this embodiment, the aperture half angle is 30 degrees. Consequently, in the stowed position as described below, the closure element 160 is well outside the liquid path and the risk of it contacting liquid flowing out of the funnel 140 is greatly reduced.

In this embodiment, the first and second retention links 170, 171 attach the connector 161 to the plug 162 and funnel 140 respectively. The retention links 170, 171 are spaced along the connector 161 so that the connector 161 is connected to the funnel 140 at one end, then to the plug 162 by the first retention link 170, then to the funnel by the second retention link 171 and then finally back to the plug 162. The connector thereby has an S shape as it connects alternatively between the funnel 140 and plug 162. In addition, the second retention link 171 is provided approximately 30-50% of the way from the outlet end 143 to the inlet portion 144 and therefore, the plug 162 when in the stowed position does not extend past the outlet end 143 of the funnel 140.

The connector 161 and second retention link 171 also connect to the funnel 140 on one side of the locator rib 146. That is, they are not over or adjacent to the locator rib 146 to ensure they do not impede identification of the locator rib 146. They are also not opposite the locator rib 146, as the locator rib 146 is intended to be facing upwards in use when the catheter is in the insertion orientation. Liquid drops could come out of the funnel 140 and drop directly onto the closure element 160 if it was position on the opposite side of the funnel 140 to the locator rib 146.

In this embodiment, the retention links 170, 171 are breakable. Thus, to move the plug 162 out of the stowed position the user can simply pull the plug 162 and funnel 140 apart to break the retention links 170, 171 as described below.

In this embodiment, the wetting mechanism 180 is provided at an end of the sleeve 110 that corresponds to the proximal end 102 of the catheter 101. The wetting mechanism 180 is tubular with a length defined along its tubular shape. The wetting mechanism 180 is configured to store a wetting agent, which in this embodiment is water, and then release it to wet the catheter 101 in use. The wetting mechanism 180 also provides a protective seal around the proximal end 102 of the catheter 101 to protect it right until the point of use, as described below.

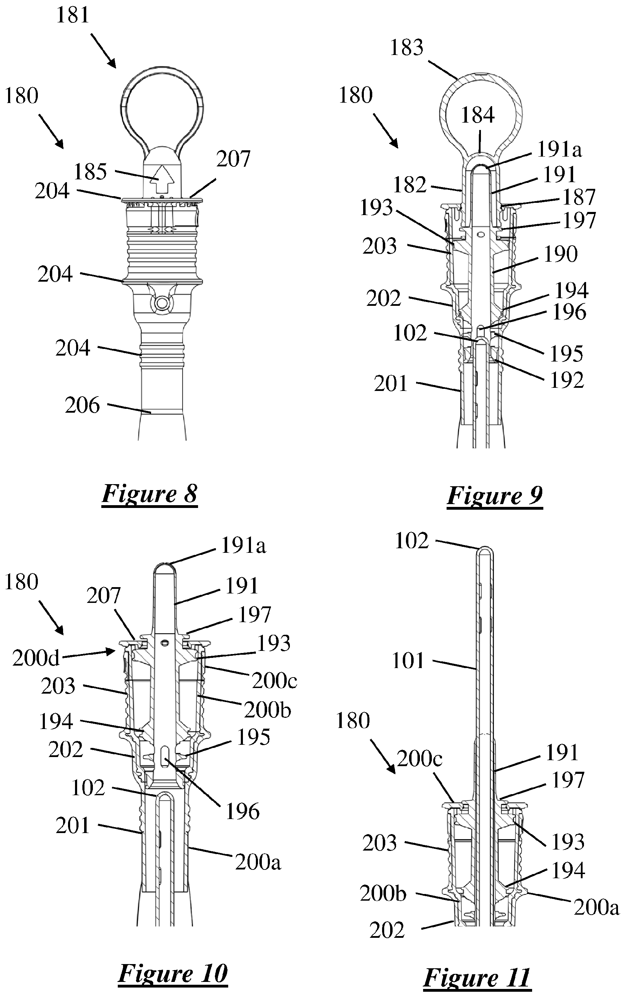

Referring to Figures 8 to 11 in particular, the wetting mechanism 180 comprises a removable cap 181, an inserter 190 and an external housing 200. The housing 200 is generally tubular with open ends. The housing 200 is coupled to the sleeve 110 at a catheter end 206 and is configured to receive the cap 181 to seal the housing 200 at an opposite cap end 207. The housing 200 comprises a neck 201 that extends from the sleeve 110 approximately one third of the length of the housing from the sleeve 110 to the cap 181. The housing 200 then comprises a divergent portion 202 covering about one quarter of the length of the housing 200 wherein the housing 200 increases in radial size. The housing 200 finally comprises a chamber wall 203 defined from the divergent portion to the cap 181.

In terms of the internal profile of the housing, the neck 201 is the narrowest part of the housing 200 and has a diameter of approximately double the diameter of the catheter 101. The chamber wall 203 is the widest part of the internal profile of the housing 200 and has a diameter of approximately double the diameter of the neck 201. The internal profile is substantially constant across each of the neck 201 and chamber wall 203 respectively. Across the majority of its length, the internal profile of the divergent portion 202 has a constant diameter that is midway between the sizes of the neck 201 and chamber wall 203. At either end of the divergent portion 202 the internal profile is tapered/chamfered to connect it to the neck 201 and chamber wall 203 respectively.

In terms of the external profile of the housing, it is generally the same as the internal profile described above. However, there are a number of gripping ribs 204 that extend outward from the neck 201 and chamber wall 203 to enable easier handling of the wetting mechanism 180. In particular, a gripping rib 204 is provided around the cap end 207 and between the chamber wall 203 and divergent portion 202.

In this embodiment, the inserter 190 is tubular and is sized such that it fits partially within housing 200. The external housing 200 and inserter 190 are concentrically arranged such that the inserter 190 is located within the external housing 200 in a radially nested configuration. The inserter 190 comprises an inserter tip 191 provided at a first end of the inserter 190 which closes the tubular body with a hemispherical end 191a, the hemispherical end 191a is provided with one or more apertures or openings such as slits (not shown) which allow the end 191a to be opened to allow passage of a catheter therethrough. The opposite end of the inserter 190 is provided with the catheter gripper 192.

In this embodiment, the catheter gripper 192 comprises a pair of gripping protrusions 195 extending outward on opposite sides of the tubular inserter 190. The gripping protrusions 195 are sized such that they fit within the internal diameter of the divergent portion 202 but are too large for the internal diameter of the neck 201. Between the gripping protrusions 195 there are provided opposing gripping slots 196 that extend along the inserter 190 adjacent to the gripping protrusions 195. The slots 196 are configured to allow the inserter 190 to flex to allow the gripping protrusions 195 to be accommodated in the neck 201, this in turn reduces the internal diameter of the inserter 190 such that it grips the proximal end 102 of the catheter 101 as described further below.

In this embodiment, the inserter 190 forms a wetting agent storage chamber with the housing 200, as described below, the inserter 190 is configured to provide the function of a sealing member that selectively seals the storage chamber to inhibit or permit release of wetting agent. Between the inserter tip 191 and catheter gripper 192 there is provided a first seal in the form of a first pair of ribs 193 and a second seal in the form of a second pair of ribs 194, the second pair 194 being closer to the catheter gripper 192. Each pair of ribs 193, 194 is configured to seal against the housing 200 such that a sealed cavity is formed between the housing 200, ribs 193, 194 and the inserter 190. Each pair of ribs 193, 194 are mounted on a support structure with generally trapezoid axial cross-section that extends from the tubular inserter 190 and each rib thereby comprises a small but effective extension from this structure. This ensures the ribs are less likely to be damaged during use.

In this embodiment, the first pair of ribs 193 are mounted so that they seal against the chamber wall 203 of the housing. The second pair of ribs 194 are mounted so that they seal against the straight part of the divergent portion 202.

Between the first pair of ribs 193 and the inserter tip 191, there is provided a pair of opposing activation tabs 197 which extend outward from the inserter 190 around only part of the circumference of the inserter 190. Further details of the tabs 197 are provided below.

In this embodiment, the cap 181 comprises a sheath 182 which is an open-ended tubular structure that is configured to enclose the inserter tip 191 and a pull ring 183. The sheath 182 comprises a closed hemispherical end 184 distal from the open end of the sheath 182, the hemispherical end 184 being sized to fit over the inserter tip 191. The pull ring 183 extends from the hemispherical end 184 and is sized to allow a user to insert their finger through the ring and pull the cap 181 off the wetting mechanism 180. The cap 181 also comprises an activation marker 185 which indicates to the user how to remove the cap 181, in this embodiment, the activation marker 185 is an arrow embossed into the cap 181.

In this embodiment, the cap 181 further comprises a pair of apertures 186 arranged to receive the activation tabs 197 of the inserter 190. The cap 181 also comprises a pair of slits (not shown) between the apertures 186 which have a similar function to the slots 196 of the inserter 190 but instead allow the apertures 186 to flex outward so that they can move over the tabs 197 to either engage or disengage from the inserter 190.

Finally, in this embodiment, the cap 181 comprises a plurality of pairs of locator bumps 187. These are provided around the circumference of the cap 181 to locate it in the cap end 207 of the housing 200 and prevent inadvertent removal of the cap 181 from the housing.

In this embodiment, the inserter 190 is arranged within the housing 200 with the tip 191 extending out of the cap end 207 and the rest of the inserter 190 within the housing 200. The inserter 190 is moveable with respect to the housing 200 between a first position in which the inserter 190 is retracted and a second position in which the inserter 190 is deployed.

In the first position, the gripping protrusions 195 are arranged in the neck 201 and therefore the catheter gripper 192 is compressed inward and grips the proximal end 102 of the catheter 101. In addition, the second pair of ribs 194 are positioned in the straight portion of the divergent portion 202 and therefore provides a seal between the inserter 190 and housing 200 at this location. The first pair of ribs 193 are positioned against the chamber wall 203 and thereby form the wetting agent storage chamber. Finally, the activation tabs 197 are within the housing 200 and are engaged by the apertures 186 of the cap 181. The cap 181 is inserted into the housing 200 such that the cap end 207 bears onto the cap 181 between the locator bumps in each pair of locator bumps 187. In this position, the cap end 207 prevents the cap 181 from flexing outwards and therefore the cap 181 can't be removed from the inserter 190 when the inserter is in the first position.

Thus, in the first position, the proximal end 102 of the catheter 100 is protected from damage and dirt by the wetting mechanism but the wetting agent is also securely stored.

In the second position, the inserter 190 is positioned differently with respect to the housing 200. The gripping protrusions 195 are now located in the divergent portion 202 and therefore the catheter gripper 192 does not grip the proximal end 102 of the catheter 100. In addition, the second pair of ribs 194 are positioned outside the divergent portion 202 and in line with the chamber wall 203. As such, a gap is formed between the second pair of ribs 194 and the chamber wall 203 and the storage chamber is opened, allowing wetting agent to flow around the ribs 194 and onto the catheter 100. Finally, the first pair of ribs 193 are provided against cap end 207 and the activation tabs 197 are positioned outside the housing 200. The activation tabs 197 are sized to engage the cap end 207 slightly and so provide resistance to movement of the inserter 190 back to the first position. In this position, the cap 181 may or may not be attached to the inserter 190.

Preferably, when the catheter assembly is in the closed configuration, the inserter is in the first position to limit further release of wetting agent, whereas, when the catheter assembly is in the open configuration, the inserter may be in the first or second positions as chosen by the user.

In this embodiment, the wetting mechanism 180 is formed of five component parts, two of which are the cap 181 and inserter 190. The housing 200 is formed from three of the component parts: a first component part 200a, a second component part 200b, and a third component part 200c. The first component part 200a is tubular and defines the internal profile of the divergent portion 202 and chamber wall 203. The second component part 200b is also tubular and is overmoulded onto the first component part 200a. The second component part forms the neck 201 and defines the external profile of the divergent portion 202 and part of the chamber wall 203. The third component part 200c forms the cap end 207 and defines part of the external profile of the chamber wall 203. The third component part 200c is snap-fit onto the first component part 200a via a locking mechanism 200d provided on the first and third component parts 200a, 200c and formed from complementary locking slots/protrusions.

To manufacture the packaged catheter assembly 1, funnel 140 is formed by injection moulding the entire funnel 140 including the closure element 160 in a single mould. This is possible due to the tapering inlet and outlet portions 144, 145 as well as the tapered ribs 152, 153. Advantageously, this also provides a closure element 160 that is integrally formed with the rest of the funnel 140. As such, it remains attached even when in the open configuration which makes it less likely the closure element 160 becomes lost.

In this embodiment, the wetting mechanism 180 is similarly formed using injection moulding. The housing 200, inserter 190 and cap 181 may all be formed separately and then joined together. The first and third component parts 200a, 200c, cap 181 and inserter 190 may be formed and then the second component part 200b overmoulded onto the first component part 200a. The wetting mechanism can then be put together, for example, the cap 181 may be fitted to the inserter 190 and then the inserter 190 and cap 181 inserted together into the first component 200a. This may be done by partially inserting the inserter 190 into the housing 200 such that the second pair of ribs 194 seals against the housing 200, then filling the storage chamber with wetting agent, for example through a small filling window (not shown), then sealing the storage chamber by moving the inserter to the first position and sealing the filling window. The cap 181 may then be fixed to the housing 200.

The catheter 101 can then be provided and coupled to the funnel 140 along with the sleeve 110. The catheter 101 can be coupled to the funnel 140 by simply pushing it into a CRZ and then sealing to the CRZ, for example with an adhesive. Where two different sizes of catheter are made in the same production process, the same funnel 140 can be used for both assuming the funnel 140 has a corresponding CRZ for each size/type of catheter. Finally, the wetting mechanism 180 can be coupled to the other end of the sleeve 110 to seal the catheter 101 inside. In this embodiment, the catheter 101 is provided spaced apart from the storage chamber along the length of the wetting mechanism. Of course in other embodiments, the catheter assembly could be constructed in a different order.

The catheter assembly 100 can then be placed within the walls 3 of the pouch 2 and the peripheral seal 4 formed to seal the catheter assembly 100 in a sterile environment within the pouch 2.

In use, the packaged catheter assembly 1 is provided to the user sealed shut. The user can then grasp the tear-away region 5 and tear it from the pouch 2 to form an opening. They can then remove the catheter assembly 100 from the pouch 2.

To access the catheter 101, the user must pull the cap 181 off the wetting mechanism 180, which they can do so using the pull ring 183. Once pulled, the cap 181 can be removed as the cap end 207 no longer prevents outward flexion of the cap around the tabs 197. In addition, the inserter 190 is automatically moved from the first position to the second position by the cap 181 engaging the activation tabs 197 of the inserter 190. This user actuation causes wetting agent to be released from the storage chamber to into the sleeve where it may effectively wet the catheter 101. This release of wetting agent occurring independently of the catheter and other components of the catheter assembly due to the design of the wetting mechanism.

The user can then hold the funnel 140 and wetting mechanism 180 and move them together to move the proximal end 102 of the catheter 101 into the wetting mechanism 180 and then through the inserter 190 and out of the inserter tip 191, then into the body. The user continues inserting the catheter into the body and uses the locator rib 146 if required to align the catheter correctly. Once the catheter reaches the bladder, liquid will flow through the catheter, into the funnel and out of the catheter assembly.

After the flow of liquid has ceased, the user may then seal the end of the funnel 140. They first break the retention links 170, 171 to move the closure element 160 out of the stowed position, the plug 162 can then be freely moved into the outlet end 143 of the funnel 140 and inserted until the cap end 164 of the closure element 160 bears against the outlet end 143 of the funnel. Any unwanted leakage of liquid from inside the catheter 101 or sleeve 110 is now avoided and the catheter assembly can be more conveniently repackaged into the pouch 2 ready for disposal.

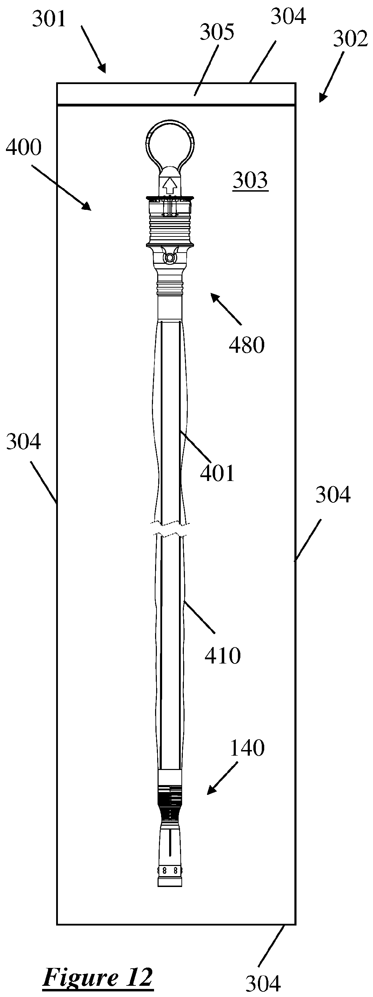

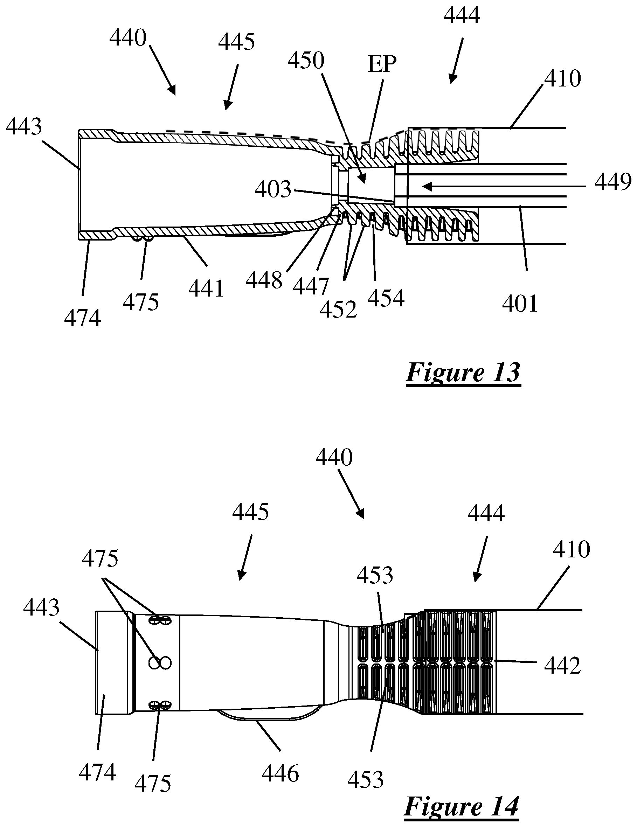

Referring to Figures 12 to 16, a second embodiment of a packaged catheter assembly 301 comprises many of the same features as the first embodiment described above. Consequently, only the differences are discussed below and the same numeral plus 300 are used for corresponding features.

The main difference between the two embodiments is the structure of the funnel and the way in which the catheter assembly avoids accidental leakage of liquid from the catheter assembly after use.

In this embodiment, the funnel 440 does not comprise a closure element. In contrast the funnel 440 is configured to fit together with the wetting mechanism 480 with a push-fit as described below. The sleeve 410, funnel 440 and wetting mechanism 480 thereby create a closed cavity around the catheter 301 to prevent accidental leakage of liquid.

In this embodiment, the funnel comprises a skirt 474 at the outlet end 443. The skirt 474 comprises a widened portion of the outlet portion 445 that is about 1 mm wider diameter than the rest of the outlet portion 445. The skirt 474 spans about 10% of the length of the funnel 440 from the outlet end 443. The skirt is configured to engage the wetting mechanism as described further below.

In this embodiment, the funnel comprises a plurality of locating protrusions in the form of six pairs of locating protrusions 475. The protrusions in each pair 475 are arranged adjacent to one another along the length of the funnel 440. The pairs 475 are all positioned at the same point along the length of the funnel, about 20% of the length of the funnel from the outlet end 443. The pairs 475 are evenly spaced around the circumference of the funnel 440. Each locating protrusion is hemispherical and extends outward from the surface of the funnel by about 0.6 mm. The locating protrusions therefore extend out past the external profile of outlet end 443 and skirt 474. Each pair of locating protrusions 475 is configured to engage the wetting mechanism, by being overridden by the wetting mechanism, as described further below to allow the funnel to be located and fit together with the wetting mechanism by a push-fit. The locator rib 446 extends axially between the locating protrusions and the inlet portion 444.

The locator bumps 487 of the cap 481 are essentially identical to the locating protrusions 475 provided on the funnel 440 and the bumps 487 and protrusions 475 perform the same role of locating the cap 481 and funnel 440 respectively in the cap end 507.

In this embodiment, the catheter assembly 400 comprises an open configuration in which liquid flow through the funnel 440 and out of the catheter assembly 400 is permitted. In this configuration, the funnel 440 is therefore not fit into the wetting mechanism 480. Instead, the cap 481 might be fit into the wetting mechanism 480 instead, for example before use.

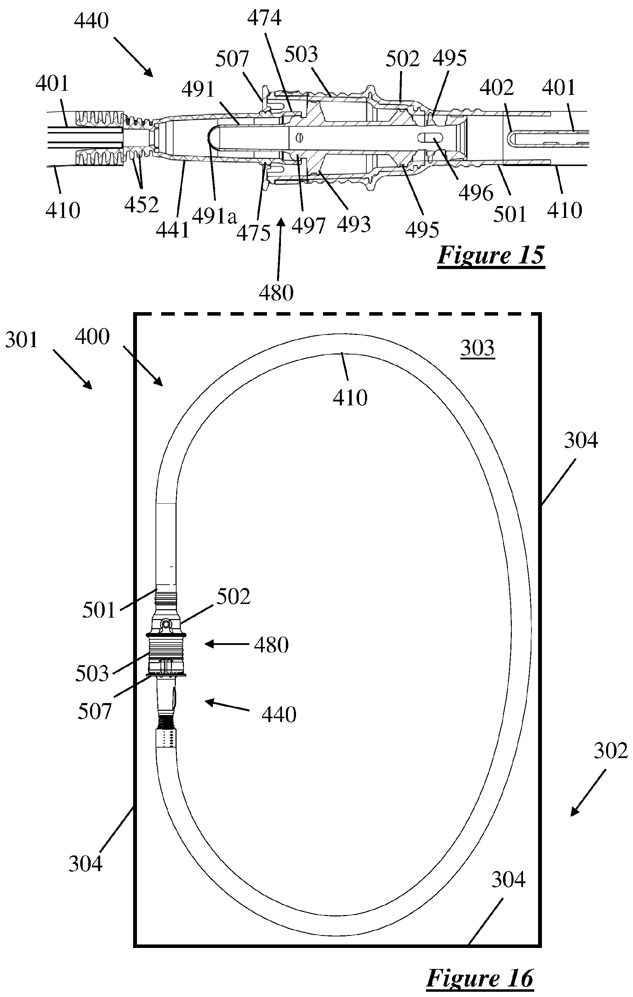

In this embodiment, as described above, the catheter 301 is contained in a cavity formed by the sleeve 410, wetting mechanism 480 and funnel 440. The catheter assembly 400 comprises a closed configuration in which the cavity containing the catheter is closed to restrict liquid flow through the funnel 440 and out of the catheter assembly 400. In the closed configuration, the funnel 440 is push-fit together with the wetting mechanism as shown in Figure 15. In this position, the inserter tip 491 is received by the funnel 440, and the skirt 474 overrides and bends the activation tabs 497, and simultaneously, the cap end 507 overrides one of each of the pair of locating protrusions 475. The internal profile of the wetting mechanism 480, specifically the cap end 507, thereby matches the external profile of the funnel 440 to enable this. Thus, the funnel 440 is seated inside the wetting mechanism 480 with a push-fit, and the inside and outside of the funnel are engaged by the wetting mechanism simultaneously. This is advantageous as it is easy and convenient for the user to do, especially if they have limited dexterity.

Additionally, when in the closed configuration, the inserter 490 is in its first position and seals against the housing 500 of the wetting mechanism 480. In this embodiment, the funnel 440 may only be push-fit with the wetting mechanism 480 when the inserter is in the first position. In this case, insertion of the funnel 440 into the wetting mechanism 480 will drive the inserter 490 into the first position if it is not already there. This helps to reduce liquid flow inside the catheter assembly and any resultant leakage.

Due to the annular shape of the cap end 507 and the support of the cap end 507 by the housing 200, the cap end 507 is structurally more rigid than the tubular funnel 440 and therefore, the funnel 440 can compress slightly to help the locating protrusions override the cap end 507 and be fitted into the wetting mechanism 480.

In this embodiment, in the closed configuration, the cavity is closed but it is not liquid-tight. Instead, the closed cavity provides a tortuous and difficult path for liquid to escape the catheter assembly. For example, liquid in the funnel 440 must pass along the funnel 440 in one direction and past the activation tabs 497, then turn around the outlet end 443 and pass back in the other direction along the outside of the funnel 440 before passing between the funnel 440/locating protrusions 475 and the cap end 507. Similarly, any liquid in the catheter 401 must first travel to the funnel 440 either via the proximal end 402 and inserter tip 491 or distal end 403 of the catheter 401 and then follow the path as described above. Therefore, the tortuous path provides the user with enough time to repackage the catheter assembly 400 into the pouch 302 as described below without a high likelihood of leaks from the catheter 401 or funnel 440.

The method of manufacture is very similar to the first embodiment except the closure element is of course not formed. In both the first and second embodiments, the catheter assembly is provided in the pouch in the open configuration. This is important as it reduces the number of actions a user needs to complete before using the catheter assembly.

In terms of use of the catheter assembly, the main difference is after the flow of liquid through the catheter has ceased. In this embodiment, the user closes the catheter inside a cavity by inserting the outlet end 443 of the funnel 440 into the cap end 507 of the wetting mechanism 480. The user keeps inserting until the locating protrusions 475 are overridden by the cap end 507 and the cap end 507 is seated between the protrusions in each pair of locating protrusions 475. This may involve using the funnel 440 to drive the inserter 490 from the second position to the first position. A tortuous liquid path is now formed to prevent unwanted leaks from inside the catheter 401 or sleeve 410 and the catheter assembly can be more conveniently repackaged into the pouch 302 ready for disposal as shown in Figure 16.

The one or more embodiments are described above by way of example only. Many variations are possible without departing from the scope of protection afforded by the appended claims.

The following numbered paragraphs additionally describe embodiments of the invention as set forth herein.
1. A catheter assembly comprising a catheter comprising a proximal end for insertion into the body and a distal end, and a funnel configured to direct liquid out of the distal end of the catheter,
   wherein the funnel comprises a tube defining an internal profile of the funnel and a plurality of projections extending outward from the tube,
   wherein each projection comprises a tip distal from the tube and the tips of the projections together define at least part of an external profile of the funnel,
   wherein the internal profile comprises a stepped region and a corresponding region of the external profile is smooth.
2. A catheter assembly according to paragraph 1 wherein the catheter is an intermittent urinary catheter, preferably wherein the catheter is an intermittent male urinary catheter.
3. A catheter assembly according to paragraph 1 or paragraph 2 wherein the stepped region comprises at least two steps in the internal profile.
4. A catheter assembly according to any one of paragraphs 1 to 3 wherein the tube has a substantially constant wall thickness.
5. A catheter assembly according to any one of paragraphs 1 to 4 wherein the internal profile comprises a step at a transition point along the length of the funnel and the projections are provided on both sides of the transition point.
6. A catheter assembly according to paragraph 5 wherein the projections extend further from the tube on one side of the transition point than the other.
7. A catheter assembly according to paragraph 5 or 6 wherein a corresponding but inverse change in the projections as compared to the internal profile is provided at the transition point.
8. A catheter assembly according to any one of paragraphs 1 to 7 wherein the projections have a radial length defined as the distance the projections extend from the internal tube in a radial direction and the radial length changes along the length of the funnel.
9. A catheter assembly according to paragraph 8 wherein changes in the radial length counter changes in the internal profile.
10. A catheter assembly according to any one of paragraphs 1 to 9 wherein the tube comprises two or more catheter receiving zones, wherein each catheter receiving zone is configured to receive a different type and/or size of catheter.
11. A catheter assembly according to paragraph 10 wherein each catheter receiving zone is configured to receive and retain the distal end of a different type and/or size of catheter.
12. A catheter assembly according to paragraph 10 or 11 wherein the two or more catheter receiving zones are arranged in series along the length of the funnel.
13. A catheter assembly according to any one of paragraphs 10 to 12 wherein the internal size/shape of the funnel changes between adjacent catheter receiving zones.
14. A catheter assembly according to paragraph 13 wherein the stepped region comprises a region spanning at least two catheter receiving zones.
15. A catheter assembly according to any one of paragraphs 10 to 14 wherein the projections are provided in a position corresponding to at least one of the two or more catheter receiving zones.
16. A catheter assembly according to any one of paragraphs 1 to 15 wherein the plurality of projections comprise a plurality of ribs.
17. A catheter assembly according to paragraph 16 wherein the plurality of ribs comprise one or more circumferential ribs extending around a circumference of the funnel.
18. A catheter assembly according to paragraph 16 or paragraph 17 wherein the plurality of ribs comprise one or more axial ribs extending along the length of the funnel.
19. A catheter assembly according to any one of paragraphs 1 to 18 further comprising a wetting mechanism, wherein the wetting mechanism and funnel are provided at either end of the sleeve and the wetting mechanism, funnel and sleeve together define a cavity containing the catheter,
   wherein the wetting mechanism comprises a storage chamber configured to retain a wetting agent, the wetting mechanism is configured to release the wetting agent to wet the catheter in use, and
   wherein the funnel and wetting mechanism fit together to close the cavity with a push-fit.
20. A catheter assembly comprising a catheter comprising a proximal end for insertion into the body and a distal end, a wetting mechanism, a funnel, and a tubular sleeve,
   wherein the wetting mechanism and funnel are provided at either end of the sleeve and the wetting mechanism, funnel and sleeve together define a cavity containing the catheter,
   wherein the wetting mechanism comprises a storage chamber configured to retain a wetting agent, the wetting mechanism is configured to release the wetting agent to wet the catheter in use, and
   wherein the funnel and wetting mechanism fit together to close the cavity with a push-fit.
21. A catheter assembly according to paragraph 19 or 20 wherein the catheter is an intermittent urinary catheter, preferably wherein the catheter is an intermittent male urinary catheter.
22. A catheter assembly according to any one of paragraphs 19 to 21 wherein the wetting mechanism is tubular with a length defined along the tubular shape of the wetting mechanism, and the proximal end of the catheter is spaced part from the storage chamber along the length of the wetting mechanism.
23. A catheter assembly according to any one of paragraphs 19 to 22 wherein the wetting mechanism comprises a bore sized to allow the catheter to pass out of the cavity.
24. A catheter assembly according to any one of paragraphs 19 to 23 wherein the wetting mechanism is configured to be user actuatable to independently release the wetting agent to wet the catheter.
25. A catheter according to any one of paragraphs 19 to 24 wherein the wetting mechanism and funnel are configured such that when push-fit together, release of wetting agent from the storage chamber is inhibited.
26. A catheter assembly according to any one of paragraphs 19 to 25 wherein the wetting mechanism is configured to simultaneously engage both an inside and an outside of the funnel when push-fit together.
27. A catheter assembly according to any one of paragraphs 19 to 26 wherein the wetting mechanism comprises an inserter, and the funnel is configured to fit around the inserter.
28. A catheter assembly according to paragraph 27 wherein the inserter comprises an inserter tip and the funnel is configured to receive the inserter tip when the funnel is push-fit together with the wetting mechanism.
29. A catheter assembly according to any one of paragraphs 19 to 28 wherein the wetting mechanism comprises one or more tabs configured to engage the funnel when push-fit with the wetting mechanism.
30. A catheter assembly according to any one of paragraph 19 to 29wherein the wetting mechanism and funnel have a different rigidity.
31. A catheter assembly according to paragraph 30wherein the funnel is less rigid than the wetting mechanism.
32. A catheter assembly according to any one of paragraph 19 to 31further comprising a cap configured to close the wetting mechanism before use.
33. A catheter assembly according to paragraph 32 wherein the cap and wetting mechanism fit together to close the end of the wetting mechanism with an interlocking mechanism.
34. A catheter assembly according to paragraph 32 or 33 wherein only one of the cap or funnel can be fit together with the wetting mechanism at a time.
35. A catheter assembly according to any one of paragraphs 19 to 34 wherein the catheter assembly comprises an open configuration in which fluid flow through the funnel and out of the catheter assembly is permitted and a closed configuration in which the cavity is closed and the funnel is push-fit into the wetting mechanism.
36. A catheter assembly according to paragraph 35 further comprising a cap configured to close the wetting mechanism before use, wherein the cap is fitted into the wetting mechanism in the open configuration.
37. A catheter assembly according to any one of paragraphs 1 to 36 wherein the funnel comprises a closure element configured to inhibit liquid flow out of the funnel,
   wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations.
38. A catheter assembly comprising a catheter comprising a proximal end for insertion into the body and a distal end,
   and a funnel configured to direct liquid out of the distal end of the catheter, the funnel comprising a closure element configured to inhibit liquid flow out of the funnel,
   wherein the catheter assembly is moveable from an open configuration in which liquid flow out of the funnel is permitted by the closure element to a closed configuration in which the closure element inhibits liquid flow out of the funnel, and
   wherein the closure element is connected to the funnel in both the open and closed configurations.
39. A catheter assembly according to paragraph 37 or 38wherein the catheter is an intermittent urinary catheter, preferably wherein the catheter is an intermittent male urinary catheter.
40. A catheter assembly according to any one of paragraph 37 to 39wherein in a stowed position, the catheter assembly is in the open configuration and the closure element does not impede liquid flow out of the funnel.
41. A catheter assembly according to paragraph 40 wherein the closure element comprises a retainer configured to retain the closure element in the stowed position.
42. A catheter assembly according to paragraph 41 wherein the retainer is breakable to release the closure element from the stowed position.
43. A catheter assembly according to any of paragraphs 40 to 42 wherein the closure element comprises a connector attached to the funnel and a closure body configured to inhibit liquid flow out of the funnel in the closed configuration, wherein the connector is substantially S-shaped in the stowed position.
44. A catheter assembly according to any one of paragraphs 37 to 43 wherein the closure element comprises a connector and a closure body, wherein the connector is attached to the funnel and the closure body is configured to inhibit liquid flow out of the funnel in the closed configuration, and the connector is flexible to allow movement of the closure body with respect to the funnel.
45. A catheter assembly according to any one of paragraphs 37 to 44 wherein the closure element comprises a plug which plugs the funnel in the closed configuration.
46. A catheter assembly according to any one of paragraphs 37 to 45 wherein the catheter is rotation dependent and comprises an insertion orientation configured to enable the catheter to release fluid from the body, wherein the closure element is arranged above or to one side of the funnel when the catheter is in the insertion orientation.
47. A catheter assembly according to any one of paragraphs 37 to 46 wherein the closure element and/or funnel comprises one or more sealing protrusions configured to provide a seal between the funnel and closure element.
48. A catheter assembly according to paragraph 47 wherein the one or more sealing protrusions comprise one or more sealing ribs
49. A catheter assembly according to paragraph 48 wherein the one or more sealing ribs preferably extend completely around the closure element in a ring shape.
50. A catheter assembly according to any one of paragraphs 37 to 49 wherein the closure element comprises a cap which caps the funnel in the closed configuration.
51. A catheter assembly according to any one of paragraphs 37 to 50 wherein the closure element and funnel are integrally formed.
52. A catheter assembly according to any one of paragraphs 37 to 51 further comprising a sleeve configured to enclose the catheter from the proximal end to the distal end.
53. A catheter assembly according to paragraph 52 further comprising a wetting mechanism configured to store a wetting agent and release the wetting agent into the sleeve to wet the catheter in use.
54. A packaged catheter assembly comprising a sterile-sealed pouch containing the catheter assembly of any one of paragraphs 1 to 53.
55. A packaged catheter assembly according to paragraph 54 wherein the catheter assembly is provided in the sterile-sealed pouch in the open configuration.
56. A packaged catheter assembly according to paragraph 54 or 55 when dependent on any one of paragraphs 19 to 36 wherein the catheter assembly comprises an open configuration in which fluid release through the funnel is permitted and a closed configuration in which the cavity is closed and the funnel is push-fit into the wetting mechanism, and
   wherein the catheter assembly is provided in the sterile-sealed pouch in the open configuration.
57. A method of manufacturing a catheter assembly comprising providing a catheter comprising a proximal end for insertion into the body and a distal end, and forming a funnel configured to direct liquid out of the distal end of the catheter, wherein the funnel comprises a tube defining an internal profile of the funnel and a plurality of projections extending outward from the tube,
   wherein each projection comprises a tip distal from the tube and the tips of the projections together define at least part of an external profile of the funnel,
   wherein the internal profile comprises a stepped region and a corresponding region of the external profile is smooth.
58. A method of manufacturing a catheter assembly, the method comprising providing a catheter, a tubular sleeve, a wetting mechanism and a funnel, attaching the wetting mechanism and funnel to either end of the sleeve, and inserting the catheter into the sleeve such that a cavity is formed around the catheter by the wetting mechanism, sleeve and funnel, and the funnel is configured to direct liquid out of the distal end of the catheter, wherein the wetting mechanism comprises a storage chamber configured to retain a wetting agent, the wetting mechanism is configured to release the wetting agent to wet the catheter in use, and
   wherein the funnel and wetting mechanism are constructed to fit together to close the cavity with a push-fit.
59. A method of manufacturing a catheter assembly comprising, providing a catheter comprising a proximal end for insertion into the body and a distal end, and forming a funnel configured to direct liquid out of the distal end of the catheter, and attaching the funnel to the distal end of the catheter, wherein the funnel comprises a closure element configured to inhibit liquid flow out of the funnel, wherein the catheter assembly is moveable from an open configuration in which liquid flow out of the funnel is permitted by the closure element to a closed configuration in which the closure element inhibits liquid flow out of the funnel, and wherein the closure element is connected to the funnel in both the open and closed configurations.
60. A method according to paragraph 59 wherein the step of forming the funnel comprises injection moulding.
61. A method according to paragraph 59 or 60 wherein the funnel and closure element are formed simultaneously.
62. A method of stowing a catheter assembly after use, the catheter assembly comprising a catheter comprising a proximal end for insertion into the body and a distal end, a tubular sleeve, a wetting mechanism, and a funnel configured to direct liquid out of the distal end of the catheter,
   wherein the wetting mechanism and funnel are provided at either end of the sleeve,
   wherein the wetting mechanism comprises a storage chamber configured to retain a wetting agent, the wetting mechanism is configured to release the wetting agent to wet the catheter in use, and
   the method comprises push-fitting the funnel and wetting mechanism together to form a closed cavity around the catheter with the sleeve.
63. A method of stowing a catheter assembly after use, the catheter assembly comprising a catheter comprising a proximal end for insertion into the body and a distal end, and a funnel configured to direct liquid out of the distal end of the catheter, the method comprising moving a closure element of the funnel from an open configuration in which liquid flow out of the funnel is permitted by the closure element to a closed configuration in which the closure element inhibits liquid flow out of the funnel., wherein the funnel and closure element are connected in both the open and closed configurations.
64. The method of any one of paragraphs 57 to 63 wherein the catheter assembly is a catheter assembly according to any of paragraphs 1 to 53.

## Claims

1. A catheter assembly comprising a catheter comprising a proximal end for insertion into the body and a distal end, a wetting mechanism, a funnel, and a tubular sleeve,
wherein the wetting mechanism and funnel are provided at either end of the sleeve and the wetting mechanism, funnel and sleeve together define a cavity containing the catheter,
wherein the wetting mechanism comprises a storage chamber configured to retain a wetting agent, the wetting mechanism is configured to release the wetting agent to wet the catheter in use, and
wherein the funnel and wetting mechanism fit together to close the cavity with a push-fit.

2. A catheter assembly according to claim 1 wherein the wetting mechanism is tubular with a length defined along the tubular shape of the wetting mechanism, and the catheter is spaced part from the storage chamber along the length of the wetting mechanism.

3. A catheter assembly according to claim 1 or claim 2 wherein the wetting mechanism is configured to simultaneously engage both an inside and an outside of the funnel when push-fit together.

4. A catheter assembly according to any one of the preceding claims wherein the wetting mechanism comprises one or more tabs configured to engage the funnel when push-fit with the wetting mechanism.

5. A catheter assembly according to any one of the preceding claims wherein the funnel comprises a plurality of locating protrusions configured to engage the wetting mechanism.

6. A catheter assembly according to claim 5 wherein the locating protrusions are provided in pairs.

7. A catheter assembly according to any one of the preceding claims further comprising a cap configured to close the wetting mechanism before use and the cap and funnel are configured to fit into the same part of the wetting mechanism.

8. A catheter assembly according to claim 7 wherein the cap and wetting mechanism fit together to close the end of the wetting mechanism with an interlocking mechanism.

9. A catheter assembly according to any one of the preceding claims wherein the catheter is an intermittent urinary catheter, preferably wherein the catheter is an intermittent male urinary catheter.

10. A catheter assembly according to any one of the preceding claims wherein the wetting mechanism is configured to be user actuatable to independently release the wetting agent to wet the catheter.

11. A catheter according to any one of the preceding claims wherein the wetting mechanism and funnel are configured such that when push-fit together, release of wetting agent from the storage chamber is inhibited.

12. A catheter assembly according to any one of the preceding claims wherein the wetting mechanism comprises an inserter, and the funnel is configured to fit around the inserter.

13. A catheter assembly according to claim 12 wherein the inserter comprises an inserter tip and the funnel is configured to receive the inserter tip when the funnel is push-fit together with the wetting mechanism.

14. A catheter assembly according to any one of the preceding claims wherein the wetting mechanism and funnel have a different rigidity and wherein the funnel is less rigid than the wetting mechanism.

15. A catheter assembly according to any one of the preceding claims wherein the catheter assembly comprises an open configuration in which fluid flow through the funnel and out of the catheter assembly is permitted and a closed configuration in which the cavity is closed and the funnel is push-fit into the wetting mechanism.
